# EUROPÄISCHE PATENTSCHRIFT

(11) **EP 3 063 188 B1**
(45) Veröffentlichungstag und Bekanntmachung des Hinweises auf die Patenterteilung: **13.12.2017**
(21) Anmeldenummer: 14790620.0
(22) Anmeldetag: 30.10.2014
(51) Int. Cl.: C04B 24/32, C07C 323/14, C04B 24/16

(54) **ALKOXYLATE VON S-VINYLTHIOALKANOLEN**
ALKOXYLATES OF S-VINYLTHIOALKANOLES
ALKOXYLATES DE S-VINYLTHIOALKANOLES

(30) Priorität: 30.10.2013 EP 13190787
(43) Veröffentlichungstag der Anmeldung: 07.09.2016
(73) Patentinhaber: BASF SE, 67056 Ludwigshafen am Rhein (DE)
(72) Erfinder: MAITRO-VOGEL, Sophie, 68199 Mannheim (DE); ERNST, Martin, 69121 Heidelberg (DE); SCHADE, Christian, 67063 Ludwigshafen (DE); TUZINA, Pavel, 68163 Mannheim (DE); TRAN-THIEN, Hoang Trang, 33102 Paderborn (DE); REIS-WALTHER, Eva-Maria, 64747 Breuberg (DE); SHABELINA, Natalia, 67063 Ludwigshafen (DE); HILLESHEIM, Nina Susanne, 63667 Nidda (DE); SCHOLZ, Christian, 84518 Wald a. d. Alz (DE)
(74) Vertreter: BASF IP Association
(86) Internationale Anmeldenummer: PCT/EP2014/073281
(87) Internationale Veröffentlichungsnummer: WO 2015/063194

(56) Entgegenhaltungen:
- WO-A1-2005/000922
- DE-A1- 2 058 120

## Beschreibung

Die vorliegende Erfindung betrifft Alkoxylate von S-Vinylthioalkanolen und Polymere, die solche Alkoxylate von S-Vinylthioalkanolen als Monomere enthalten. Gegenstand der Erfindung sind weiterhin Verfahren zur Herstellung von Polymeren enthaltend Alkoxylate von S-Vinylthioalkanolen als Monomere. Weitere Gegenstände der Erfindung sind die Verwendungen solcher Polymere und Mischungen enthaltend solche Polymere.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils konkret angegebenen Kombination, sondern auch in anderen Kombinationen verwendbar sind, ohne den Rahmen der Erfindung zu verlassen. Bevorzugt beziehungsweise ganz bevorzugt sind insbesondere auch diejenigen Ausführungsformen der vorliegenden Erfindung, in denen alle Merkmale des erfindungsgemäßen Gegenstandes die bevorzugten beziehungsweise ganz bevorzugten Bedeutungen haben.

Die WO 93/06142 A1 betrifft Copolymerisate aus Hydroxyalkylvinylethern, Additionsprodukten von C2- bis C4-Alkylenoxiden an Hydroxyalkylvinylether und/oder Polytetrahydrofuranvinylether und optional anderen copolymerisierbaren Monomeren.

In der WO 94/04580 A1 werden Copolymerisate beschrieben, die hergestellt werden durch Polymerisation von wasserlöslichen, ethylenisch ungesättigten säuregruppenhaltigen Monomeren und Monomeren, welche eine Polyalkoxysequenz enthalten. Die Copolymerisate können weitere Monomere enthalten.

WO 02/066528 A2 beschreibt alkoxylierte Acrylat- und Methacrylat-Makromonomere, die als Additive in Beton eingesetzt werden können. Copolymere der Makromonomere mit anderen Monomeren wie Acrylsäure, Methacrylsäure oder Maleinsäure sind ebenfalls in der WO 02/066528 A2 erwähnt.

In der WO 2005/000922 A1 wird ein Dispersionsmittel für Zementzusammensetzungen beschrieben, welches als Monomere unter anderem alkoxylierte Allylalkoholsulfate und optional weitere alkoxylierte Allylalkoholgruppen enthält.

Die WO 2005/005500 A1 betrifft wasserlösliche oder in Wasser dispergierbare Polymerisate enthaltend alkoxylierte Diallylaminderivate, ethylenisch ungesättigte Mono- oder Dicarbonsäuren, deren Anhydride oder Gemische davon sowie gegebenenfalls eines oder mehrere weitere ethylenisch ungesättigte Monomere. Diese Polymerisate finden unter anderem Verwendung als Additive in mineralischen Baustoffen.

WO 2009/040042 A1 beschreibt Polycarboxylatether, die sich für einen Einsatz als Betonverflüssiger oder Dispergiermittel für anorganische Pigmente eignen.

DE 20 58 120 offenbart 2-Hydroxyethylvinylsulfid bzw. Derivate davon umfassende kationische Polymere, welche mittels eines zweistufigen Verfahrens hergestellt werden. In einem ersten Schritt wird 2-Hydroxyethylvinylsulfid, ggf. zusammen mit Comonomeren wie Acrylaten polymerisiert. Die erhaltenen (Co)polymere werden in einem zweiten Schritt mit Alkylierungsmitteln umgesetzt, wobei die S-Atome unter Bildung von Sulfoniumgruppen alkyliert werden. Als Alkylierungsmittel können Alkylsulfate wie Dimethylsulfat, Alkylhalogenide oder Alkylenoxide verwendet werden, wobei bei der Verwendung von Alkylenoxiden anorganische oder organische Säuren in äquimolaren Mengen eingesetzt werden müssen.

Teilweise treten bei den Polymeren des Standes der Technik Probleme bei der Herstellung auf. Beispielsweise zeigen Allylalkoholethoxylate bei der Polymerisation oder Copolymerisation keine besonders hohe Reaktivität und führen auch zu einigen Nebenreaktionen. Isoprenolethoxylate sind ebenfalls nicht sehr reaktiv und beim Herstellprozess kann es zur Entstehung von Isopren kommen. Einige der Alkoxylate, beispielsweise Hydroxybutylvinylether-ethoxylate (HBVE-ethoxylate) sind insbesondere im sauren Milieu nur bedingt hydrolysestabil.

Aufgabe der vorliegenden Erfindung war es Polymere bereit zu stellen, die die oben genannten Nachteile nicht aufweisen. Eine Teilaufgabe der Erfindung war es für eine effiziente Polymerisationsreaktion reaktive Alkoxylatmonomere zu entwickeln. Als eine weitere Teilaufgabe der vorliegenden Erfindung sollten solche Alkoxylatmonomere bereitgestellt werden, die sowohl als Monomer, als auch einpolymerisiert in das Polymer eine erhöhte Hydrolysestabilität aufweisen.

Diese und andere Aufgaben werden, wie aus dem Offenbarungsgehalt der vorliegenden Erfindung ersichtlich, durch die verschiedenen Ausführungsformen der Erfindung gelöst, insbesondere durch ungesättigte Verbindungen der allgemeinen Formel (I) 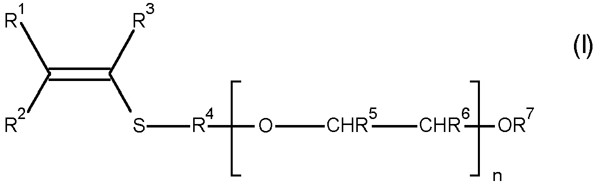 wobei
- R¹, R², R³: unabhängig voneinander, gleich oder verschieden, H, CH₃, bevorzugt H,
- R4: lineares oder verzweigtes C₁-C₃₀-Alkylen, bevorzugt C₂-C₁₀-Alkylen, besonders bevorzugt C₂-C₄-Alkylen, insbesondere -CH₂-CH₂-,
- R⁵, R⁶: unabhängig voneinander, gleich oder verschieden, H, C₁-C₂₀-Alkyl, C₃-C₁₅-Cycloalkyl, Aryl,
CH₂-O-C₁-C₂₀-Alkyl, CH₂-O-C₂-C₂₀-Alkenyl, wobei R⁵ und R⁶ auch gemeinsam auch ein C₃-C₆-Alkylen bilden, bevorzugt Tetramethylen bilden können, bevorzugt H, -CH₃, -CH₂-CH₃, -C₃-C₁₁-Alkyl, C₁₂-C₂₂-Alkyl, Phenyl, CH₂-O-C₁-C₁₀-Alkyl, CH₂-O-C₂-C₁₀-Alkenyl,
besonders bevorzugt H, -CH₃, -CH2-O-CH2-CH=CH2, -CH₂-O-2-Ethylhexyl, insbesondere bevorzugt H oder -CH₃ und ganz besonders bevorzugt H, R⁷ unabhängig voneinander, gleich oder verschieden, H, C₁-C₄-Alkyl oder 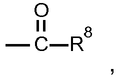 bevorzugt H oder C₁-C₄-Alkyl, besonders bevorzugt H,
- R⁸: C₁-C₂₂-Alkyl, C₂-C₂₂-Alkenyl,
bevorzugt C₁₂-C₁₈-Alkyl, C₁₂-C₁₈-Alkenyl,
- n: ganze Zahl von 2 bis 200, insbesondere von 2 bis 160,
bevorzugt von 5 bis 140, besonders bevorzugt 10 bis 80 und beispielsweise 20 bis 30 beträgt
bedeuten.

Ausdrücke der Form Cₐ-C_{b} bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form Cₐ-C_{b}-V. V steht hierbei für eine chemische Verbindungsklasse oder Substituentenklasse, beispielsweise für Alkylverbindungen oder Alkylsubstituenten.

Im Einzelnen haben die für die verschiedenen Substituenten angegebenen Sammelbegriffe folgende Bedeutung:
C₁-C₂₂-Alkyl: geradkettige oder verzweigte Kohlenwasserstoffreste mit bis zu 22 Kohlenstoffatomen, beispielsweise C₁-C₁₀-Alkyl oder C₁₁-C₂₂-Alkyl, bevorzugt C₁-C₁₀-Alkyl beispielsweise C₁-C₃-Alkyl, wie Methyl, Ethyl, Propyl, Isopropyl, oder C₄-C₆-Alkyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methyl-pentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Trimethylpropyl, 1-Ethyl-1-methylpropyl, 1-Ethyl-2-methylpropyl, oder C₇-C₁₀-Alkyl, wie Heptyl, Octyl, 2-Ethyl-hexyl, 2,4,4-Trimethylpentyl, 1,1,3,3-Tetramethylbutyl, Nonyl oder Decyl sowie deren Isomere.

C₂-C₂₀-Alkenyl: ungesättigte, geradkettige oder verzweigte Kohlenwasserstoffreste mit 2 bis 20 Kohlenstoffatomen und einer, zwei oder drei, bevorzugt einer Doppelbindungen in einer beliebigen Position, beispielsweise C₂-C₁₀-Alkenyl oder C₁₁-C₂₀-Alkenyl, bevorzugt C₂-C₁₀-Alkenyl wie C₂-C₄-Alkenyl, wie Ethenyl, 1-Propenyl, 2-Propenyl, 1-Methylethenyl, 1-Bute-nyl, 2-Butenyl, 3-Butenyl, 1-Methyl-1-propenyl, 2-Methyl-1-propenyl, 1-Methyl-2-pro-penyl, 2-Methyl-2-propenyl, oder C₅-C₆-Alkenyl, wie 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl oder 1-Ethyl-2-methyl-2-propenyl, sowie C₇-C₁₀-Alkenyl, wie die Isomere von Heptenyl, Octenyl, Nonenyl oder Decenyl.

C₁-C₃₀-Alkylen: geradkettige oder verzweigte Kohlenwasserstoffreste mit 1 bis 30 Kohlenstoffatomen, beispielsweise C₁-C₁₀-Alkylen oder C₁₁-C₂₀-Alkylen, bevorzugt C₁-C₁₀-Alkylen, insbesondere Methylen, Dimethylen, Trimethylen, Tetramethylen, Pentamethylen oder Hexamethylen.

C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl oder Cyclooctyl sowie ein gesättigtes oder ungesättigtes cyclisches System wie z. B. Norbornyl oder Norbenyl.

Aryl: ein ein- bis dreikerniges aromatisches Ringsystem enthaltend 6 bis 14 Kohlenstoffringglieder, z. B. Phenyl, Naphthyl oder Anthracenyl, bevorzugt ein ein- bis zweikerniges, besonders bevorzugt ein einkerniges aromatisches Ringsystem.

In einer bevorzugten Ausführungsform der Erfindung handelt es sich bei den ungesättigten Verbindungen (I) um solche der allgemeinen Formel (la)

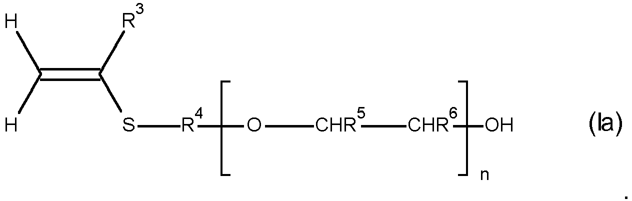

In der Formel (la) stehen R³ für H oder Methyl, bevorzugt H, R⁴ für eine lineare oder verzweigte C₂-C₁₀-Alkylengruppe, bevorzugt eine lineare C₂-C₁₀-Gruppe, insbesondere eine lineare oder verzweigte, bevorzugt eine lineare C₂-C₄-Alkylengruppe. Beispiele umfassen 1,2-Ethylen-, 1,3-Propylen- und 1,4-Butylengruppen, und ganz besonders bevorzugt handelt es sich bei R⁴ um eine 1,2-Ethylengruppe -CH₂CH₂-.

Bei der Gruppe -[-O-CHR⁵⁻CHR⁶⁻]ₙ,- in Formel (la) handelt es sich um eine Polyalkoxygruppe umfassend n Alkoxygruppen -O-CHR⁵-CHR⁶-, wobei die Alkoxygruppen jeweils gleich oder verschieden sein können. Bei R⁵ und R⁶ handelt es sich unabhängig voneinander um H, Methyl oder Ethyl, bevorzugt H oder Methyl und ganz besonders H, mit der Maßgabe, dass die Summe der Kohlenstoffatome in den Resten R⁵ und R⁶ pro Alkoxygruppe jeweils 0 bis 2 beträgt. Mit anderen Worten gesagt, umfasst die Polyalkoxygruppe also Gruppen ausgewählt aus der Gruppen von Ethoxy-, Propoxy- und Butoxygruppen. Sofern verschiedene Alkoxygruppen anwesend sind, können diese in beliebiger Reihenfolge angeordnet sein, beispielsweise statistisch, alternieren oder blockweise. In einer bevorzugten Ausführungsform handelt es sich bei mindestens 50 mol %, bevorzugt bei mindestens 80% der Alkoxygruppen um Ethyoxygruppen. Besonders bevorzugt handelt es sich ausschließlich um Ethoxygruppen, d.h. R⁵ und R⁶ sind H.

In Formel (la) steht n für eine Zahl von 2 bis 200, bevorzugt 5 bis 160, besonders bevorzugt 10 bis 140, ganz besonders bevorzugt 20 bis 140 und beispielsweise 20 bis 30.

In einer weiteren bevorzugten Ausführungsform der Erfindung handelt es sich bei den ungesättigten Verbindungen (I) um solche der allgemeinen Formel (Ib) 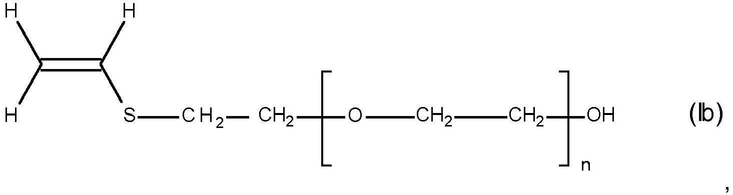 wobei n für eine Zahl von 2 bis 200, bevorzugt 5 bis 160, besonders bevorzugt 10 bis 140, ganz besonders bevorzugt 20 bis 140 und beispielsweise 20 bis 30 steht.

Die Herstellung der Verbindungen (I), insbesondere der Verbindungen der Formel (la) kann insbesondere durch Alkoxylierung von ungesättigten Verbindungen der allgemeinen Formel (II) 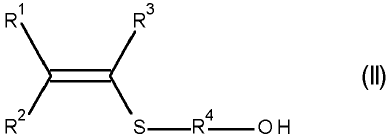 erfolgen. Hierzu setzt man die Verbindung (II) mit der gewünschten Menge von Alkylenoxiden bzw. Alkylenetheroxiden, insbesondere C₂- bis C₄-Alkylenoxiden, besonders bevorzugt Ethylenoxid um.

Die Durchführung einer Alkoxylierung ist dem Fachmann prinzipiell bekannt. Hierbei empfiehlt es sich regelmäßig, Säuren als Katalysator zur Alkoxylierung zu vermeiden. In einer bevorzugten Ausführungsform der Erfindung handelt sich bei der Alkoxylierung um eine basenkatalysierte Alkoxylierung. Dazu kann die als Ausgangsmaterial verwendete Verbindung (II) in einem Druckreaktor mit basischen Katalysatoren, insbesondere Alkalihydroxiden, bevorzugt Kaliumhydroxid oder mit Alkalialkoholaten wie beispielsweise Kaliummethylat versetzt werden.

Die Alkoxylierung kann aber auch mittels anderer Methoden vorgenommen werden. Beispielsweise können Doppelhydroxidtone, wie in DE 4325237 A1 beschrieben, eingesetzt werden oder es können Doppelmetallcyanid-Katalysatoren (DMC-Katalysatoren) verwendet werden. Geeignete DMC-Katalysatoren sind beispielsweise in der DE 10243361 A1, insbesondere in den Abschnitten [0029] bis [0041] sowie der dort zitierten Literatur, offenbart. Beispielsweise können Katalysatoren vom Zn-Co-Typ eingesetzt werden. Zur Durchführung der Reaktion kann der Alkohol (R¹)(R²)-CH-CH₂-OH mit dem Katalysator versetzt, die Mischung wie oben beschrieben entwässert und mit den Alkylenoxiden wie beschrieben umgesetzt werden. Es werden üblicherweise nicht mehr als 1000 ppm Katalysator bezüglich der Mischung eingesetzt und der Katalysator kann aufgrund dieser geringen Menge im Produkt verbleiben. Die Katalysatormenge kann in der Regel geringer sein als 1.000 ppm, beispielsweise 250 ppm oder weniger.

Ein weiterer Gegenstand der vorliegenden Erfindung sind Mischungen, enthaltend Verbindungen der allgemeinen Formel (I), bevorzugt der allgemeinen Formel (la), besonders bevorzugt der allgemeinen Formel (Ib). Die Menge an Verbindungen der allgemeinen Formel (I) in solchen Mischungen kann, abhängig vom Verwendungszweck der Mischungen, über einen weiten Bereich variieren. Die Menge an Verbindungen der allgemeinen Formel (I) in solchen Mischungen beträgt in der Regel von 1 bis 99 Gew.-%, bevorzugt von 10 bis 95 Gew.-%, bezogen auf die Gesamtmenge der Mischung.

Bei derartigen Mischungen kann es sich beispielsweise um Mischungen verschiedener Verbindungen der allgemeinen Formel (I), bevorzugt der allgemeinen Formel (la), besonders bevorzugt der allgemeinen Formel (Ib) untereinander handeln. Weiterhin kann es sich beispielsweise um Mischungen mit Lösemitteln, anderen Monomeren und/oder weiteren Additiven wie beispielsweise Stabilisatoren zur Verhinderung der Polymerisation, Tensiden und/oder anderen Zusatzstoffen und Additiven handeln.

In einer bevorzugten Ausführungsform der Erfindung handelt es sich bei solchen Mischungen, um wässrige Lösungen der Verbindungen der allgemeinen Formel (I). Ein Vorteil solcher wässriger Lösungen der Verbindungen der allgemeinen Formel (I) ist eine leichte Handhabbarkeit, insbesondere bei der Dosierung.

Ein weiterer Gegenstand der vorliegenden Erfindung sind Polymere, enthaltend Verbindungen der allgemeinen Formel (I), bevorzugt solchen der Formel (la), besonders bevorzugt solchen der allgemeinen Formel (Ib)als Monomere. Es kann sich um Homopolymere der Verbindungen (I) oder Verbindungen (I) umfassende Copolymere handeln.

In einer bevorzugten Ausführungsform der Erfindung umfassen die erfindungsgemäßen Polymere mindestens ein weiteres von den Verbindungen der allgemeinen Formel (I) verschiedenes Monomer (weiteres Monomer). Selbstverständlich können auch mehrere von den Verbindungen der allgemeinen Formel (I) verschiedene Monomere im Polymer enthalten sein.

Besonders bevorzugt handelt es sich bei dem mindestens einem weiteren Monomeren um ein monoethylenisch ungesättigtes Monomer.

Geeignete weitere Monomere sind C₂-C₂₄ Alkene, beispielsweise Ethen, Propen, 1-Buten, 2-Buten, Isobuten, Diisobuten, 1-Hexen, 1-Hepten, 1-Octen, 1-Nonen, 1-Decen, 1-Dodecen, 1-Octadecen.

Geeignete weitere Monomere sind auch konjugierte C₄-C₁₀ Diene, beispielsweise Butadien, Isopren oder Chloropren.

Bei weiteren Monomeren, insbesondere monoethylenisch ungesättigten weiteren Monomeren kann es sich insbesondere um Säuregruppen umfassende Monomere handeln, wobei die Säuregruppen auch ganz oder teilweise neutralisiert sein können. Es kann sich hierbei insbesondere um Alkalimetallsalze, Erdalkalimetallsalze, Ammoniumsalze oder Salze organischer Ammoniumionen handeln. Bevorzugt kann es sich um Monomere umfassend Säuregruppen ausgewählt aus der Gruppen von Carbonsäure-, Sulfonsäure-, Phophorsäure- oder Phosphonsäuregruppen handeln.

Beispiele geeigneter weiterer Monomere mit Carbonsäuregruppen umfassen C₃-C₁₂ monoethylenisch ungesättigte Mono- oder Dicarbonsäuren, ihre Anhydride oder Salze wie Acrylsäure, Methacrylsäure, (Meth)acrylsäureanhydrid, Crotonsäure, Maleinsäure, Maleinsäureanhydrid, Fumarsäure, Itaconsäure, Mesaconsäure, Citraconsäure oder Methylenmalonsäure und ihre Ammonium- oder Alkalisalze. Die Säuren können ganz oder teilweise in neutralisierter Form eingesetzt werden.

Beispiele geeigneter weiterer Monomere mit Phosphorsäure- bzw. Phosphonsäuregruppen umfassen monoethylenisch ungesättigte Phosphonsäure- oder (Poly)phosphorsäureester und ihre Salze wie Vinylphosphonsäure oder Ester von Hydroxyethyl, Hydroxypropyl- oder Hydroxybutyl(meth)acrylat mit (Poly)phosphorsäure und ihre Alkali und Ammoniumsalze, Phosphorsäuremonovinylester, Allylphosphonsäure, Phosphorsäuremonoallylester, 3-Butenylphosphonsäure, Phosphorsäure(mono-3-butenyl)ester, Phosphorsäuremono-(4-vinyl-oxybutyl)ester, Phosphorsäuremono-(-2-hydroxy-3-vinyloxy-propyl)ester, Phosphorsäuremono-(1-phosphonoxymethyl-2-vinyloxy-ethyl)-ester, Phosphorsäuremono-(3-allyloxy-2-hydroxypropyl)ester, Phosphorsäuremono-2-(allylox-1-phosphonoxymethyl-ethyl)ester, 2-Hydroxy-4-vinyloxymethyl-1,3,2-dioxaphosphol, 2-Hydroxy-4-allyloxymethyl-1,3,2-dioxaphosphol. Es können auch Salze und/oder Ester, insbesondere C₁-C₈-Mono-, Di- sowie gegebenenfalls Trialkylester der Phosphorsäure und/oder Phosphonsäuregruppen aufweisenden Monomere eingesetzt werden.

Beispiel geeigneter weiterer Monomere mit Sulfonsäuregruppen umfassen monoethylenisch ungesättigte Sulfonsäuren und ihre Salze wie Vinylsulfonsäure, 2-Acrylamido-2-methylpropanesulfonsäure, 2-Acrylamidomethyldodecylsulfonsäure, 2-(Meth)acryloxyethansulfonsäure, 3-(Meth)acryloxypropansulfonsäure, Allyloxybenzolsulfonsäure, Vinylbenzolsulfonsäure, Vinyltoluolsulfonsäure, Allylsulfonsäure, Methallylsulfonsäure und ihre entsprechenden Ammonium- und Alkalimetallsalze.

Geeignete weitere Monomere sind auch Ester, Amide und Imide monoethylenisch ungesättigter Mono- oder Dicarbonsäuren, insbesondere der oben genannten monoethylenisch ungesättigten C3-C12 Carbonsäuren, insbesondere C₁-C₄₀, bevorzugt C₁-C₂₂, besonders bevorzugt C₂-C₁₂ Ester, Amide oder ImideDie Substituenten können dabei auch weitere Heteroatome tragen. Dicarbonsäuren können dabei auch in Form ihrer Halbester oder Halbamide vorliegen, beispielsweise als C₁ - C₄ Halbester. Die Amide und Imide können in N-mono- oder ggf. N,N-dialkylierter Form vorliegen.

Bei Estern kann es sich insbesondere um Ester von (Meth)acrylsäure handeln, insbesondere (Meth)acrylsäureester mit aliphatischen oder cycloaliphatischen Estergruppen, insbesondere C₁-C₂₂, bevorzugt C₂-C₁₂ Estergruppen handeln. Beispiele solcher Verbindungen umfassen Methyl(meth)acrylat, Ethyl(meth)acrylat, Propyl(meth)acrylat, Isopropyl(meth)acrylate, 1-Butyl (meth)acrylate, Isobutyl(meth)acrylat, tert.-Butyl(meth)acrylat, Pentyl(meth)acrylat, Isoamyl(meth)acrylat, Hexyl(meth)acrylat, Heptyl(meth)acrylate, iso-Decyl(meth)acrylat, Lauryl(meth)acrylate, Stearyl(meth)acrylat, Behenyl(meth)acrylat, Cyclohexyl(meth)acrylat, 4-tert.-Butyl-cyclohexyl(meth)acrylat, iso-Bornyl(meth)acrylat, 2-Ethylhexyl(meth)acrylat, 2-Propylheptyl(meth)acrylat oder Citronellol(meth)acrylat.

Die Estergruppen können auch Hetereoatome, insbesondere O- und/oder N-Atome umfassen. Beispiele derartiger Ester umfassen Hydroxyethyl(meth)acrylat, Hydroxypropyl(meth)acrylat, Hydroxybutyl(meth)acrylat, Ethyldiglycol(meth)acrylat, Hydroxypropylcarbamat(meth)acrylat, Phenyl(meth)acrylat, Benzyl(meth)acrylat, 2-Phenylethyl(meth)acrylat, 3-Phenylpropyl(meth)acrylat, Ureido(meth)acrylat, Acetoacetoxyethyl(meth)acrylat, Hydroxyethylpyrrolidon(meth)acrylat, tert.-Butylaminoethyl(meth)acrylat, Diethylaminoethyl(meth)acrylat, Dimethylaminoethyl(meth)acrylat. Beispiele bevorzugter Ester von (Meth)acrylsäure umfassen Hydroxyethyl(meth)acrylat, Hydroxypropyl(meth)acrylat sowie Hydroxybutyl(meth)acrylat.

Bei den Alkoholkomponenten in den (Meth)acrylestern kann es sich auch um alkoxylierte Alkohole handeln. Zu nennen sind hier insbesondere alkoxylierte C₁-C₁₈-Alkohole, welche 2 bis 80 Mol Ethylenoxid, Propylenoxid, Butylenoxid oder Mischungen davon aufweisen. Beispiele derartiger alkoxylierter Produkte umfassen Methylpolyglykol(meth)acrylat oder (Meth)acrylsäureester von mit 3, 5, 7, 10 oder 30 Mol Ethylenoxid umgesetztem C₁₃/C₁₅-Oxoalkohol bzw. deren Mischungen.

Weitere Beispielevon Estern, Amiden oder Imiden umfassen Maleinsäuremonoethylester, Maleinsäurediethylester, Dimethylmaleat, N-substituierte Maleinimide wie N-Methyl-, N-Phenyl- und N-Cyclohexylmaleinimid, Acrylamid, Methacrylamid, N-Methyl(meth)acrylamid, N,N-Dimethyl(meth)acrylamid, N,N-Diethylacrylamid, N-isopropyl(meth)acrylamid, N-Methylol(-meth)acrylamid, N-Hydroxyethyl(meth)acrylamid, N-tert.-Butyl(meth)acrylamid, N-tert.-Octyl(meth)acrylamid, N-(1-Methylundecyl)(meth)acrylamid, 10-Acrylamidoundecansäure, N-Cyclohexyl(meth)acrylamid, Diacetonacrylamid, Dimethylaminoethyl(meth)acrylamid, Dimethylaminopropyl(meth)acrylamid, N,N-Dimethyl-N-(meth)acrylamidopropyl-N-(3-sulfopropyl)ammonium-betain, (Meth)Acryloylmorpholin.

Monomere, die Amino- oder Iminogruppen tragen können auch protoniert oder in Form ihrer quaternisierten Salze vorliegen, beispielsweise durch Quaternisierung mit Methylchlorid, Dimethylsulfat oder Diethylsulfat. Die Monomere können auch mit Propansulton zu den entsprechenden Betainen umgesetzt vorliegen.

Ebenfalls geeignete weitere Monomere sind N-Vinylgruppen enthaltende Monomere, beispielsweise N-Vinylpyrrolidon, N-Vinylcaprolactam, N-Vinyl-N-methylacetamid, N-Vinylimidazol, 2-Methyl-1-vinylimidazol, quaternisierte N-Vinylimidazolderivate, beispielsweise 1-Vinyl-3-methyl-imidazolium Chlorid oder Methosulfat, N-Vinyl-1,2,4-triazol, N-Vinylcarbazol, N-Vinylformamid, 2-Methyl-1 -vinylimidazolin.

Geeignete weitere Monomere sind C₁-C₂₄ Ester von Vinylalkohol und Monocarbonsäuren, beispielsweise Vinylformiat, Vinylacetate, Vinylpropionate, Vinyl-n-butyrate, Vinyllaurate, Vinylstearate, oder Vinylester von Kochschen Säuren, beispielsweise von 2,2-Dimethylpropansäure, 2,2-Dimethylbutansäure, 2,2-Dimethylpentansäure, 2-Ethyl-2-Methylbutansäure, Neononansäure, Neodecansäure.

Weiterhin geeignet sind Vinyl- oder Allylether wie z.B. Methylvinylether, Ethylvinylether, Propy-Ivinylether, Isobutylvinylether, tert.-Butylvinylether, 2-Ethylhexylvinylether, Vinylcyclohexylether, Vinyl-4-hydroxybutylether, Decylvinylether, Dodecylvinylether, Octadecylvinylether, Hydroxybutylvinylether, 2-(Diethylamino)ethylvinylether, 2-(Di-n-butyl-amino)ethylvinylether oder Methyldiglykolvinylether bzw. die entsprechenden Allylverbindungen.

Weiterhin geeignet sind ungesättigte Alkohole wie 3-Buten-1-ol, 2-Buten-1-ol, Allyllakohol, Isoprenol, Prenol, Methallylalkohol.

Weiterhin geeignet sind alkoxylierte Vinyl-, Allyl-, Methallyl- oder Isoprenylether mit 1-150 mol EO- Einheiten oder Gemischen von EO- und PO-Einheiten.

Geeignete weitere Monomere sind ebenfalls N-Allyl-Verbindungen, beispielsweise Diallylamin, N,N-dimethyl-N,N-diallylammonium chloride.

Geeignete weitere Monomere sind auch α,β-monoethylenisch ungesättigte Nitrile mit 3 to 10 Kohlenstoffatomen, beispielsweise Acrylnitril, Methacrylnitril, Fumanitril, Maleonitril.

Geeignete weitere Monomere sind weiterhin vinylaromatische Monomere wie Styrol, Vinyltoluol oder α-Methylstyrol. Weitere Styrolderivate genügen der allgemeinen Formel IV 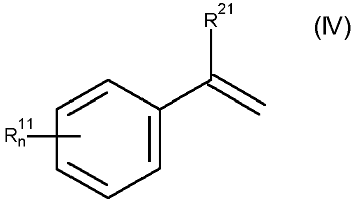 in der R¹¹ und R²¹ für Wasserstoff oder C₁-C₈-Alkyl stehen und n gleich 0, 1, 2 oder 3 ist. Der aromatische Ring kann weiterhin Heteroatome tragen beispielsweise 2- und 4-Vinylpyridin.

Geeignete weitere Monomere sind weiterhin halogenierte Alkene, beispielsweise Vinylchlorid, Vinylidenchlorid, Trifluorethylen, Tetrafluorethylen, sowie Acrolein, Methacrolein.

Bei den weiteren Monomeren kann es sich auch um vernetzend wirkende Monomere handeln. Beispiele für geeignete vernetzende weitere Monomere umfassen Moleküle mit mehreren ethylenisch ungesättigten Gruppen, beispielsweise Di(meth)acrylate wie Ethylenglykol- di(meth)acrylat, Butandiol-1,4-di(meth)acrylat oder Hexandioldi(meth)acrylat oder Poly(meth)acrylate wie Trimethylolpropantri(meth)acrylat, Pentaerythritoltri(meth)acrylat oder auch Di(meth)acrylate von Oligo- oder Polyalkylenglykolen wie Di-, Tri- oder Tetraethylen- oder - propylen-glykoldi(meth)acrylat. Weitere Beispiele umfassen Divinylbenzol, Divinylethylenharnstoff, Vinyl(meth)acrylat, Allyl(meth)acrylat, Isoprenyl(meth)acrylat, Prenyl(meth)acrylat, Dihydrodicyclopentadienylacrylat, Dicyclopentadienyl(meth)acrylat oder Butandioldivinylether. Geeignet sind ebenfalls Di- und Oligo-Allyl- oder Vinylether von Polyhydroxyverbindungen, beispielsweise Ethylenglycoldivinylether, Butandioldivinylether, 1,4-Cyclohexandimethanoldivinylether, Diethylenglycoldivinylether, Triethylenglycoldivinylether, Pentaerythrittri- oder tetraallylether. Geeignet sind ebenfalls Oligo-Allylamine, beispielsweise Triallylamin oder Tetraallylammoniumchlorid. Geeignet sind ebenfalls Di- und Oligo-Allylester von Polycarbonsäure, beispielsweise Diallylphthalat, Diallylmaleat, Triallyltrimellitat, Divinylester von Dicarbonsäuren wie der Bernsteinsäure und Adipinsäure. Geeignet sind ebenfalls Di-, Tri- oder Oligo(meth)acrylamide, beispielsweise N,N'-Methylenbis(meth)acrylamid. Der Gehalt an vernetzenden Monomeren beträgt in der Regel 0 bis 20 Mol-%, bezogen auf die Gesamtzahl aller Monomere, bevorzugt von 0 bis 10 Mol-% und insbesondere bevorzugt von 0 bis 5 Mol-%, und ganz besonders bevorzugt umfassen die erfindungsgemäßen Polymere keine vernetzenden Monomere.

Beispiele bevorzugter monoethylenisch ungesättigter weiterer Monomere umfassen Styrol, Butadien, Methyl(meth)acrylat, Ethylacrylat, Dibutylmaleat, Metyhl-alpha-cyanacrylat, Acrylnitril, Acrylsäure, Methacrylsäure, Maleinsäure(anhydrid), Itaconsäure, Vinylphosphonsäure, N-Vinylpyrrolidon, N,N-Dimetyl-N,N-diallylammoniumchlorid, Acrylamid, Vinylimidazol, , Vinylacetat, Allylsulfonsäure, 2-Acrylamido-2-methylpropan-sulfonsäure, insbesondere bevorzugt sind Acrylsäure, Methacrylsäure, Methyl(meth)acrylat, Maleinsäure(anhydrid), (Iso)prenylalkoxylat, (Meth)allylalkoxylat oder Hydroxybutylvinyletheralkoxylat. Ganz besonders bevorzugt sind Acrylsäure, Methacrylsäure, (Meth)acrylat, Maleinsäure(anhydrid), (Iso)prenylalkoxylat, (Meth)allylalkoxylat oder Hydroxybutylvinyletheralkoxylat.

In einer weiteren bevorzugten Ausführungsform der erfindungsgemäßen Polymere ist als mindestens ein weiteres von den Verbindungen der allgemeinen Formel (I) verschiedenes Monomer Acrylsäure im Polymer enthalten. Besonders bevorzugt sind in diesem Fall neben ungesättigten Verbindungen der allgemeinen Formel (I) und Acrylsäure keine weiteren Monomere enthalten.

In einer weiteren bevorzugten Ausführungsform der erfindungsgemäßen Polymere ist als mindestens ein weiteres von den Verbindungen der allgemeinen Formel (I) verschiedenes Monomer Methacrylsäure im Polymer enthalten. Besonders bevorzugt sind in diesem Fall neben ungesättigten Verbindungen der allgemeinen Formel (I) und Methacrylsäure keine weiteren Monomere enthalten. Die erfindungsgemäßen Verbindungen der allgemeinen Formel (I) lassen sich im Gegensatz zu den entsprechenden Vinyletherverbindungen gut mit (Meth)acrylsäure und Derivaten davon copolymerisieren.

In einer weiteren bevorzugten Ausführungsform der erfindungsgemäßen Polymere ist als mindestens ein weiteres von den Verbindungen der allgemeinen Formel (I) verschiedenes Monomer Maleinsäure(anhydrid) im Polymer enthalten. Besonders bevorzugt sind in diesem Fall neben ungesättigten Verbindungen der allgemeinen Formel (I) und Maleinsäure(anhydrid) keine weiteren Monomere enthalten.

Die erfindungsgemäßen Polymere können, selbst wenn sie nur aus bestimmten Verbindungen der allgemeinen Formel (I) als Monomere und/oder weiteren von den Verbindungen der allgemeinen Formel (I) verschiedenes Monomeren hergestellt werden, dennoch auf Grund ihrer Herstellungsweise selbstverständlich geringe Mengen an Startern oder Reglern enthalten.

In weiteren bevorzugten Ausführungsformen der erfindungsgemäßen Polymere sind neben ungesättigten Verbindungen der allgemeinen Formel (I), Acrylsäure und Methacrylsäure oder Acrylsäure und Maleinsäure(anhydrid) oder Methacrylsäure und Maleinsäure(anhydrid) keine weiteren Monomere enthalten.

In einer weiteren bevorzugten Ausführungsform der erfindungsgemäßen Polymere sind neben ungesättigten Verbindungen der allgemeinen Formel (I), Acrylsäure, Methacrylsäure und Maleinsäure(anhydrid) keine weiteren Monomere enthalten.

Die Zusammensetzung der erfindungsgemäßen Polymere kann, abhängig vom jeweiligen Verwendungszweck, über einen weiten Bereich variieren. Der Fachmann trifft eine geeignete Auswahl.

In der Regel umfassen die erfindungsgemäßen Polymere 1 bis 99,9 Gew.-% ungesättigte Verbindungen der allgemeinen Formel (I), insbesondere 5 bis 99,9 Gew.-% bevorzugt 10 bis 99,9 Gew.-%, insbesondere bevorzugt 30 bis 99,5 Gew.-%, besonders bevorzugt 50 bis 99 Gew.-% und ganz besonders bevorzugt 55 bis 96 Gew.-%, jeweils bezogen auf die Gesamtmenge an Monomeren im Polymer.

In einer bevorzugten Ausführungsform der Erfindung umfassen die erfindungsgemäßen Polymere von 1 bis 99,9 Gew.-% ungesättigte Verbindungen der allgemeinen Formel (I), bevorzugt Verbindungen (la), besonders bevorzugt Verbindungen (Ib) sowie 99 bis 0,1 Gew.-% von monoethylenisch ungesättigten weiteren Monomeren, bevorzugt 10 bis 99,9 Gew.-% Verbindungen (I) sowie 90 bis 0,1 Gew.-% von monoethylenisch ungesättigten weiteren Monomeren, besonders bevorzugt von 30 bis 99,9 Gew.-% Verbindungen (I) sowie 70 bis 0,1 Gew.-% von monoethylenisch ungesättigten weiteren Monomeren, jeweils bezogen auf die Gesamtmenge an Monomeren enthalten, mit der Maßgabe, dass die Gesamtmenge an Verbindungen der Formel (I) sowie monoethylenisch ungesättigten weiteren Monomeren mindestens 80 Gew. %, bevorzugt mindestens 90 Gew. %, besonders bevorzugt mindestens 95 Gew. % beträgt. Ganz besonders bevorzugt sind neben Verbindungen der Formel (I) sowie monoethylenisch ungesättigten weiteren Monomeren keine anderen Monomere enthalten.

In einer weiteren bevorzugten Ausführungsform umfassen die erfindungsgemäßen Polymere von 1 bis 99,9 Gew.-%, bevorzugt von 10 bis 99,9 Gew.-%, besonders bevorzugt von 30 bis 99,9 Gew.-% ungesättigte Verbindungen der allgemeinen Formel (I), bevorzugt Verbindungen (la), besonders bevorzugt Verbindungen (Ib) und insgesamt von 99 bis 0,1 Gew.-%, bevorzugt von 90 bis 0,1 Gew.-%, besonders bevorzugt 70 bis 0,1 Gew.-% weitere Monomere, wobei die genannten Mengen jeweils auf die Gesamtmenge an Monomeren im Polymer bezogen sind, und wobei es sich bei den weiteren Monomeren um mindestens eines ausgewählt aus der Gruppe von
(1) monoethylenisch ungesättigten, Carbonsäuregruppen umfassenden Monomeren bzw. deren deren Anhydride oder Salze, wie beispielsweise Acrylsäure, Methacrylsäure, Maleinsäure oder Maleinsäureanhydrid,
(2) monoethylenisch ungesättigten, Phosphor oder Phosphonsäuregruppen umfassenden Monomeren bzw. deren Salze, wie beispielsweise Ester von Hydroxyethyl, Hydroxypropyl- oder Hydroxybutyl(meth)acrylat mit (Poly)phosphorsäure,
(3) monoethylenisch ungesättigten, Sulfonsäuregruppen umfassenden Monomeren bzw. deren Salze, wie beispielsweise Vinylsulfonsäure oder 2-Acrylamido-2-methylpropanesulfonsäure,
(4) (Meth)acrylsäurehydroxyalkylestern, wie beispielsweise Hydroxyethylacrylat oder Hydroxypropylactylat,
(5) Polyalkoxygruppen umfassende, monoethylenisch ungesättigte Monomere, wie beispielsweise (Iso)prenylalkoxylat, (Meth)allylalkoxylat, Hydroxybutylvinyletheralkoxylat oder (Meth)acrylsäurealkoxylate
handelt.

In einer weiteren bevorzugten Ausführungsform umfasst das erfindungsgemäße Polymer neben mindestens einer Verbindung (I), bevorzugt (la), besonders bevorzugt (Ib) mindestens zwei verschiedene weitere Monomere, insbesondere mindestens zwei verschiedene weitere monoethylenisch ungesättigte Monomere. Bevorzugt handelt es sich bei den weiteren Monomeren um mindestens ein Säuregruppen umfassendes, monoethylenisch ungesättigtes Monomer, bevorzugt ein Monomer ausgewählt aus den oben beschriebenen Gruppen (1), (2) und (3) sowie ein OH-Gruppen und/oder Polyalkoxygruppen umfassendes monoethylenisch ungesättigtes Monomer, bevorzugt ein Monomer ausgewählt aus den oben beschriebenen Gruppen (4) und (5). In dieser bevorzugten Ausführungsform beträgt die Menge der Verbindungen (I), bevorzugt Verbindungen (la), besonders bevorzugt Verbindungen (Ib) in der Regel 1 bis 99,9 Gew.-%, bevorzugt von 10 bis 99,9 Gew.-%, besonders bevorzugt von 30 bis 99,9 Gew.-% und die Menge der beiden weiteren Monomere zusammen 99 bis 0,1 Gew.-%, bevorzugt von 90 bis 0,1 Gew.-%, besonders bevorzugt 70 bis 0,1 Gew.-%, jeweils bezogen auf die Gesamtmenge aller Monomere im Polymer.

In einer bevorzugten Ausführungsform der Erfindung umfasst das erfindungsgemäße Polymer 70 bis 99 Gew.-%, bevorzugt 80 bis 98 Gew.-% mindestens einer Verbindung (Ib) sowie 1 bis 30 Gew.-%, bevorzugt 2 bis 20 Gew. % (Meth)acrylsäure.

Die erfindungsgemäßen Polymere weisen, abhängig von der Zusammensetzung, Molmassenverteilungen auf, die über einen weiten Bereich variieren können. Der Fachmann wählt je nach dem gewünschten Anwendungszweck des Polymers eine geeignete Molmasse. Die zahlenmittlere Molmasse Mₙ kann beispielsweise 1000 g/mol bis 1000000 g/mol betragen.

Insbesondere weisen die erfindungsgemäßen Polymere eine Molmassenverteilung (Molekulargewichtsverteilung) mit einem Zahlenmittel Mₙ von 1000 bis 200000 g/mol auf, bevorzugt von 2000 bis 180000 g/mol, besonders bevorzugt von 3000 bis 150000 g/mol, insbesondere von 5000 bis 100000 g/mol und beispielsweise 10000 g/mol bis 50000 g/mol.

Ein weiterer Gegenstand der Erfindung sind Verfahren zur Herstellung von Polymeren. Die Herstellung kann insbesondere mittels radikalischer Polymerisation ungesättigten Verbindungen der allgemeinen Formel (I), bevorzugt (la), besonders bevorzugt (Ib) sowie optional weiteren Monomeren erfolgen. Verfahren zur radikalischen Polymerisation von Monomeren sind dem Fachmann prinzipiell bekannt.

Die radikalische Polymerisation kann in Masse oder bevorzugt in Lösung erfolgen. Bei der Polymerisation in Lösung richtet sich die Wahl des Lösemittels nach der Art der ungesättigten Verbindungen (I) sowie optional weiterer Monomere, insbesondere nach der Hydrophilie der Monomere. Die Polymerisation kann inbesondere in polaren Lösemitteln, bevorzugt in wässriger Lösung erfolgen. Bevorzugt können wässrige Lösungen eingesetzt werden, bei denen das verwendete Lösungsmittel bzw. Lösemittelgemisch mindestens 50 Gew. % Wasser umfasst. Daneben können weitere, mit Wasser mischbare Lösemittel vorhanden sein, z.B. Alkohole. Bevorzugt umfasst ein wässriges Lösemittel mindestens 70 Gew.-% Wasser, besonders bevorzugt mindestens 90 Gew.-% Wasser. Beispielsweise kann ausschließlich in Wasser gearbeitet werden.

Zum Starten der Polymerisation werden in prinzipiell bekannter Art und Weise Initiatoren für die radikalische Polymerisation verwendet hierbei kann es sich insbesondere um thermische Polymerisationsinitiatoren handeln, beispielsweise Peroxide oder Azoinitiatoren. Die Polymerisationstemperatur wird vom Fachmann je nach dem gewünschten Ergebnis gewählt. Bewährt hat sich eine Temperatur von 50°C bis 100°C, insbesondere bei der Polymerisation in wässriger Lösung. Die radikalische Polymerisation kann aber auch mittels anderer Techniken vorgenommen werden; es kann sich beispielsweise um eine Fotopolymerisation unter Verwendung Fotoinitiatoren handeln.

Der pH-Wert im Zuge der Polymerisation in wässriger Lösung kann vom Fachmann je nach dem gewünschten Ergebnis gewählt werden. Die erfindungsgemäßen Verbindungen (I) sind auch im sauren Bereich hydrolysestabil. Dies unterscheidet sie von den analogen, im Stand der Technik bekannten Vinyletherverbindungen H₂C=CH-O-R-(AO)ₓ, welche im sauren Bereich, insbesondere bei pH-Werten unterhalb von 3 zu Hydrolyse neigen. Dies verringert ihre Einsatzmöglichkeiten deutlich. Besonders vorteilhaft können die erfindungsgemäßen Verbindungen (I), optional zusammen mit weiteren Monomeren radikalisch in wässriger Lösung im sauren pH-Bereich polymerisiert werden, insbesondere bei pH 1 bis 6, bevorzugt 1 bis 5 und insbesondere pH 1 bis 3.

Die radikalische Polymerisation kann beispielsweise im Batch-Verfahren, Semi-Batch-Verfahren oder mittels eines kontinuierlichen Verfahrens durchgeführt werden. Ein geeignetes kontinuierliches Verfahren ist beispielsweise in der WO 2009/100956 A2 beschrieben.

Bei der radikalischen Polymerisation von ungesättigten Verbindungen (I), bevorzugt Verbindungen (la), besonders bevorzugt Verbindungen (Ib) mit weiteren Monomeren, insbesondere mit Säuregruppen umfassenden weiteren Monomeren wie beispielsweise Acrylsäure in wässriger Lösung können verschiedene Techniken eingesetzt werden.

In einer Ausführungsform der Erfindung legt man eine Mischung der Monomere, als solche oder in Lösung im Reaktionsgefäß vor und startet danach die Polymerisation, beispielsweise durch Zugabe eines thermischen Polymerisationsinitiators und Erhöhung der Temperatur.
In einer weiteren Ausführungsform der Erfindung legt man eine Lösung der ungesättigten Verbindungen (I), sowie optional einen Teil der weiteren Monomere und einen Teil eines thermischen Polymerisationsinitiators im Reaktionsgefäß vor. Es sollten bei dieser Ausführungsform nicht mehr als 25 Gew.-% der weiteren Monomere vorgelegt werden. Die verbliebene Menge weiterer Monomere sowie die verbliebene Menge des Initiators werden nach dem Start der Polymerisation, insbesondere nach Erwärmen auf Polymerisationstemperatur zugegeben. Bevorzugt werden hierbei eine Lösung weiterer Monomere und eine Lösung des Initiators kontinuierlich in das Reaktionsgefäß eindosiert.

In einer bevorzugten Ausführungsform der Erfindung dosiert man die ungesättigten Verbindungen (I) sowie die weiteren Monomere nach und nach in den Polymerisationsreaktor ein, welcher mindestens eine gewisse Menge Lösungsmittel, insbesondere ein wässriges Lösemittel enthält. Bei dieser Ausführungsform werden nur ein Teil der ungesättigten Verbindungen (I), der weiteren Monomere sowie des Initiators im Reaktionsgefäß vorgelegt, wobei die Menge der vorgelegten Monomere 25 Gew.-% der Gesamtmenge, bevorzugt 10 Gew.-% der vorgesehenen Gesamtmenge der Monomere nicht überschreiten sollte, und wobei weiterhin das Molverhältnis der vorgelegten Monomere entsprechend dem im Polymer vorgesehenen Verhältnis gewählt werden sollte. Die Abweichung sollte in der Regel nicht mehr als +/- 20 %, bevorzugt nicht mehr als +/- 10 % des vorgesehenen Verhältnis betragen. Besonders bevorzugt entspricht das Verhältnis vorgelegter Monomere dem gewünschten Monomerenverhältnis.

Die Polymerisation der vorgelegten Anteile der Monomere wird zunächst gestartet. Dies kann erfolgen, indem man den Ansatz auf die gewünschte Polymerisationstemperatur erwärmt. Alternativ kann man einen Initiator zusetzen, der die Polymerisation schon bei Raumtemperatur startet, z.B. einen Redox-Initiator. Die Polymerisation startet dann, wenn der Initiator zu den Monomeren zugegeben wird. Nach dem Start werden die die ungesättigten Verbindungen (I) und die weiteren Monomere zudosiert, bevorzugt als Lösungen. Die Monomere können hierbei separat zudosiert werden, oder man kann auch eine Mischungen aus ungesättigten Verbindungen (I) sowie weiteren Monomeren, bevorzugt eine Lösung von ungesättigten Verbindungen (I) sowie weiteren Monomeren in einem geeigneten Lösemittel zudosieren. In letzterem Falle ist das Verhältnis der ungesättigten Verbindungen (I) zu den weiteren Monomeren naturgemäß festgelegt, während im ersteren Falle das Verhältnis während der Polymerisation auch variiert werden kann. Der Initiator wird ebenfalls als Lösung in einem geeigneten Lösemittel eindosiert.

Die Dosiergeschwindigkeit bei der Zugabe der ungesättigten Verbindungen (I) sowie der weiteren Monomere sollte hierbei jeweils so gewählt werden, dass ein zu großer Überschuss nicht polymerisierter ungesättigter Verbindungen (I) bzw. nicht polymerisierter weiterer Monomere im Reaktionsgefäß vermieden wird. Insbesondere sollte ein Überschuss nicht polymerisierter ungesättigter Verbindungen (I) vermieden werden. Das Molverhältnis von ungesättigten Verbindungen (I) / weiteren Monomeren soll nachfolgend als x bezeichnet werden. Die Dosiergeschwindigkeiten der Monomere sollten bevorzugt so gewählt werden, dass das Molverhältnis der in den Reaktor einlaufenden Monomere um nicht mehr als +/- 20 %, bevorzugt nicht mehr als +/- 10 % des vorgesehenen Verhältnis abweicht, wobei selbstverständlich die Gesamtmenge der Monomere dem gewünschten Wert entsprechen muss.

Die beschriebene Ausführungsform der Polymerisation führt zu Copolymeren mit besonders guten anwendungstechnischen Eigenschaften. Die Vorteile sind besonders deutlich bei der Copolymerisation von ungesättigten Verbindungen (I) mit weiteren Säuregruppen aufweisenden weiteren Monomeren in wässriger Lösung, wie beispielsweise Acrylsäure. Dies wird im Beispielteil näher dargestellt. Ohne dass wir damit auf eine bestimmte Theorie festgelegt werden möchten, scheint der Effekt darauf zurückzuführen zu sein, dass bei der bevorzugten Ausführungsform die Monomere besonders gleichmäßig eingebaut werden.

Ein weiterer Gegenstand der Erfindung sind Verfahren zur Herstellung von Polymeren, wobei
a. eine radikalische Polymerisation von Monomeren enthaltend ungesättigte Verbindungen der allgemeinen Formel (II) und 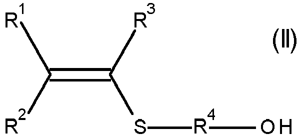
b. eine polymeranaloge Umsetzung eines in Schritt a. entstandenen Polymeren mit Verbindungen der allgemeinen Formel (III) 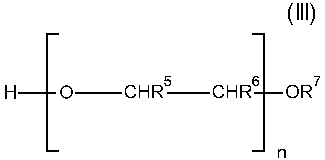
erfolgt, wobei die Symbole und Indices die oben angegebenen Bedeutungen haben.

Ein weiterer Gegenstand der vorliegenden Erfindung ist die Verwendung von erfindungsgemäßen Polymeren als Zementadditive, Mahlhilfe bei der Herstellung von Zement, Zusatz zu hydraulischen Bindemitteln, Betonverflüssiger, reaktive Weichmacher für die Herstellung von Kunststoffen (wie Polyvinylalkohol, Polyvinylbutyraldehyd), Kautschuk oder Latex (wie Styrol/Butadien Rubber - SBR, Nitril/Butadien Rubber - NBR), Assoziativverdicker, Antioxidantien, oder für die Herstellung von Polyethersiloxanen.

Ein weiterer Gegenstand der vorliegenden Erfindung sind Mischungen enthaltend erfindungsgemäße Polymere.

Bevorzugt werden die erfindungsgemäßen Polymere als Betonverflüssiger eingesetzt, bevorzugt in Mischungen , die zur Verflüssigung von Beton dienen. Solche Mischungen enthalten in der Regel ein Fließmittel vom Typ Polycarboxylatether, optional in Kombination mit einem Betonverflüssiger vom Typ Ligninsulfonat und/oder Betanaphthalinsulfonsäure-Formaldehyd-Cocondensat, Entlüfter, Luftporenbildner, Beschleuniger oder Verzögerer sowie Wasser.

Für die Anwendung als Betonverflüssiger einigen sich insbesondere Copolymere umfassend ungesättigte Verbindungen (I) sowie mindestens ein weiteres Monomer, insbesondere mindestens ein monoethylenisch ungesättigtes weiteres Monomer. Bei den ungesättigten Verbindungen (I) für diese Anwendung handelt es sich bevorzugt um Verbindungen (la), besonders bevorzugt um Verbindungen (Ib), wobei n in Formel (Ib) bevorzugt für 20 bis 140, insbesondere 20 bis 60 und ganz besonders bevorzugt 20 bis 30 beträgt. Beispiele besonders geeigneter weiterer Monomere für die Anwendung als Betonverflüssiger umfassen Acrylsäure, Methacrylsäure, Maleinsäureanhydrid, Hydroxyethyl(meth)acrylat, Hydroxypropyl(meth)acrylat, Hydroxyethyl(meth)acrylat-Phosphat, Hydroxypropyl(meth)acrylat-Phosphat.

Geeignete Copolymere für die Anwendung als Betonverflüssiger umfassen in der Regel 55 bis 99,9 Gew.-% ungesättigter Verbindungen (I) sowie 0,1 bis 45 Gew.-% monoethylenisch ungesättigter weiterer Monomere, wobei die Gesamtmenge ungesättigter Verbindungen (I) sowie monoethylenisch ungesättigter weiterer Monomere, insbesondere der soeben genannten, mindestens 90 Gew. %, besonders bevorzugt mindestens 95 Gew. % beträgt. Ganz besonders bevorzugt sind neben Verbindungen der Formel (I) sowie monoethylenisch ungesättigten weiteren Monomeren keine anderen Monomere enthalten. Das zahlenmittlere Molekulargewicht Mₙ beträgt für diese Anwendung bevorzugt 1000 g/mol bis 100000 g/mol, insbesondere 5000 g/mol bis 100000 g/mol und beispielsweise 10000 g/mol bis 40000 g/mol.

Weitere bevorzugte Mischungen sind die Mischungen der erfindungsgemäßen Polymere mit anderen Polymeren, bevorzugt Elastomeren. Beispiele solcher Polymere sind Kautschuk, Polyvinylalkohol, Polyvinylbutyral, Polyvinylchlorid, Polyvinylbutyraldehyd.

Bevorzugt enthalten, wenn als Polymere Kautschuke gewählt werden, solche Kautschuk Kautschuksorten gewählt aus der Gruppe bestehend aus SBR-Kautschuk, beispielsweise S-SBR-Kautschuk oder E-SBR-Kautschuk, BR-Kautschuk, EPM-Kautschuk, EPDM-Kautschuk, SB-Kautschuk, IIR-Kautschuk, NBR-Kautschuk und CR-Kautschuk, bevorzugt SBR-Kautschuk, NBR-Kautschuk.

Die vorliegende Erfindung stellt reaktive hydrolysestabile reaktive Alkoxylatmonomere bereit, die sich effizient zu hydrolysestabilen Polymeren umsetzen lassen.

Die Erfindung wird durch die Beispiele näher erläutert ohne dass die Beispiele den Gegenstand der Erfindung einschränken.

### Beispiele:

VME: Vinylmercaptoethanol H₂C=CH-S-CH₂CH₂OH
HBVE: Hydroxybutylvinylether H₂C=CH-O-CH₂CH₂CH₂CH₂OH

### Meßmethoden:

### OH-Zahl:

Die OH-Zahl (OHZ) ist ein Maß für die in einer Probe enthaltenen Alkoholgruppen. Die Bestimmung erfolgt, indem die Alkoholgruppen mit einem Überschuss Essigsäureanhydrid verestert werden. Nach Hydrolyse des nicht umgesetzten EssigsäuraAnhydrids wird die verbleibende freie Essigsäure mit KOH titriert. Die Angabe der OH-Zahl erfolgt in Einheiten von mg KOH / g Probe. Die OH- Zahl entspricht also der Menge KOH in "mg", die der bei der Acetylierung von 1 g Substanz gebundenen Menge Essigsäure äquivalent ist.

### PEG-Gehalt:

Als Polyethylenglykol-Gehalt gilt der dünnschichtchromatographisch bestimmbare Anteil an PEG in nichtionischen Tensiden und EO-Addukten. Er wird in g pro 100 Gramm angegeben. Polyethylenglykol wird dünnschichtchromatographisch mit Hilfe einer HPTLC-Fertigplatte vom Oxethylat in einem Substanzfleck abgetrennt und nach Derivatisation mit Dragendoff's Reagenz durch Remissionsortskurvenmessung quantifiziert. Die Auswertung erfolgt nach Eichen mit einem PEG-Standard (PEG 1000).

### GPC-Messung

### (für alle Versuche außer Versuchen 5a bis 5 g sowie Vergleichsversuchen 5a bis 5c):

### Säulen:

Folgende Säulen der Fa. PSS Mainz wurden hintereinander geschaltet:
- PSS Suprema Vorsäule
- PSS Suprema 30 Å, 10µ 8×300 mm
- PSS Suprema 1000 Å, 10µ 8×300 mm
- PSS Suprema 3000 Å, 10µ 8×300 mm
und auf 35°C temperiert.

### Säulenmaterial:

Modifiziertes Acrylatcopolymer-Netzwerk.

### Eluent:

Phosphat-Puffer pH 9,7,
0,0239 mol/L Dinatriumhydrogenphosphat(dihydrat),
pH 9,7 mit NaOH 2 mol/L eingestellt,
0,5 g/L Natriumazid.

Die Proben werden mit dem Eluenten verdünnt.

### Geräte / Software:

Agilent 1100-System mit ChemStation, Auswertung mit PSS WinGPC Unity.

### Bedingungen:

Probenverdünnung:
1,5 mL Eluent + 10 µL Aceton (interner Standard) + 20 µL Probe (bis max. 50%ige Lösung, bei höherer Konzentration weniger),
Injektion: 50 µL, mit Nadelwäsche,
Fluss: 0,8 mL/min,
Laufzeit: 60 min,
Detektor: RID (Agilent G1362A), 35°C.

### Kalibrierung:

Kalibrierstandards von PSS: Polyethylenglkol (PEG), Polyethylenoxid (PEO) für hohe Molmassen. Interner Standard zur Kompensation von Flussabweichungen: Aceton.

### GPC-Methode für die Versuche 5a bis 5 g sowie Vergleichsversuche 5a bis 5c (siehe Tabelle 4):

:Säulenkombinationen: OH-Pak SB-G, OH-Peak SB 804 HQ und OH-Pak SB 802.5 HQ von Shodex, Japan; Elutionsmittel: 80 Vol.-% wässrige Lösung von HCO₂NH₄ (0,05 mol/l) und 20 Vol.-% Acetonitril; Injektionsvolumen 100 µl; Durchflussrate 0,5 ml/min). Die Kalibrierung zur Bestimmung der mittleren Molmasse erfolgte mit linearen Polyethylenglykol-Standards. Als Maß für den Umsatz wird der Peak des Polymers auf eine relative Höhe von 1 normiert und die Höhe des Peaks des nichtumgesetzten Makromonomer/PEG-haltigen Oligomers als Maß für den Restmonomerengehalt verwendet.

### Beispiel 1: Herstellung von Alkoxylaten von S-Vinylthioalkanolen

### Beispiel 1a:

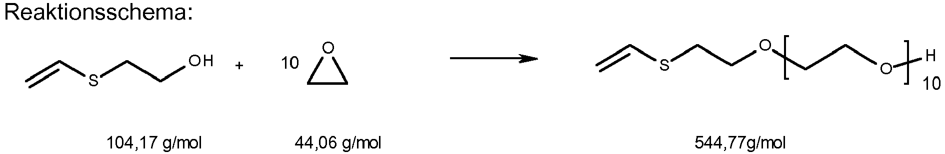

104 g (1,00 mol) Vinylmercaptoethanol und 2,18 g (19,4 mmol KOH) einer wässrigen Kaliumhydroxidlösung (50 gew.-%ig) wurden bei 70 °C in einem 2 L Autoklaven vorgelegt und bei <20 mbar innerhalb 2 h entwässert. Der Autoklav wurde anschließend mit Stickstoff geflutet und die Temperatur auf 130 °C erhöht. 440 g (10,0 mol) Ethylenoxid wurden innerhalb von 4 h zugegeben. Das erhaltene Reaktionsgemisch wurde 10 h bei 130 °C nachgerührt und nach Abkühlen auf 100 °C am Vakuum von flüchtigen Bestandteilen befreit.
Es wurden 572 g einer braunen Flüssigkeit erhalten.
OHZ = 102,5 mg KOH/g (Theorie 103,0 mg KOH/g)

### Beispiel 1b:

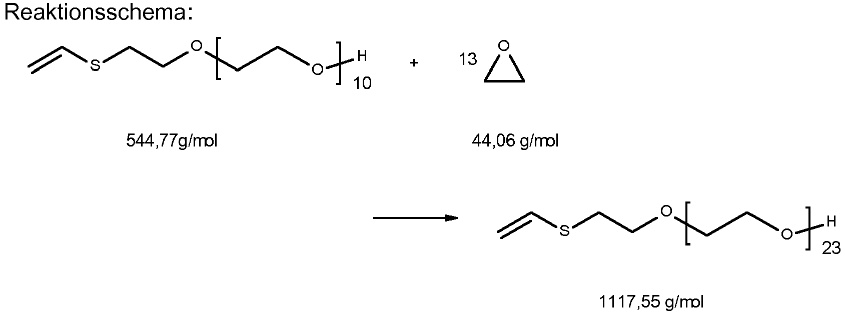

164 g (0,30 mol) Vinylmercaptoethanol*10EO (Beispiel 1a) und 686 mg (6,11 mmol KOH) einer wässrigen Kaliumhydroxidlösung (50 Gew.-%ig) wurden bei 100 °C in einem 2 L Autoklaven vorgelegt und bei <20 mbar innerhalb 2 h entwässert. Der Autoklav wurde anschließend mit Stickstoff geflutet und die Temperatur auf 130 °C erhöht. 172 g (3,90 mol) Ethylenoxid wurden innerhalb von 1,5 h zugegeben. Das erhaltene Reaktionsgemisch wurde 8 h bei 130 °C nachgerührt und nach Abkühlen auf 100 °C am Vakuum von flüchtigen Bestandteilen befreit.
Es wurden 337 g eines braunen Feststoffes erhalten.
OHZ = 55,6 mg KOH/g (Theorie 50,2 mg KOH/g); PEG-Gehalt = 10,5 Gew%

### Beispiel 1c:

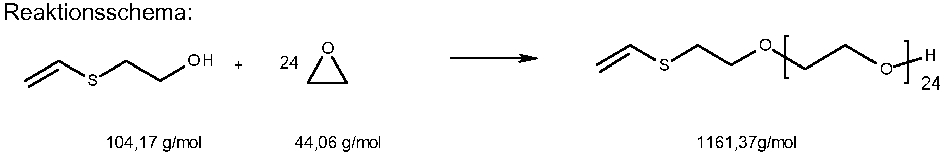

52,1 g (0,50 mol) Vinylmercaptoethanol und 410 mg (1,87 mmol KOMe) einer methanolischen Kaliummethylatlösung (32 gew.-%ig in Methanol) wurden bei 65 °C in einem 2 L Autoklaven vorgelegt und das Methanol bei <20 mbar innerhalb 2 h entfernt. Der Autoklav wurde anschließend mit Stickstoff geflutet und die Temperatur auf 120 °C erhöht. 528 g (12,0 mol) Ethylenoxid wurden innerhalb von 4 h zugegeben. Das erhaltene Reaktionsgemisch wurde 10 h bei 120 °C nachgerührt und nach Abkühlen auf 100 °C am Vakuum von flüchtigen Bestandteilen befreit.
Es wurden 606 g eines braunen Feststoffes erhalten.
OHZ = 47,5 mg KOH/g (Theorie 48,8 mg KOH/g), PEG-Gehalt: < 1 Gew%

### Beispiel 1d:

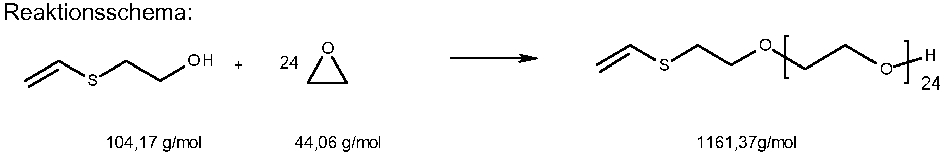

104 g (1,00 mol) Vinylmercaptoethanol und 289 mg (90 %ig, 4,50 mmol KOH) KOH-Schuppen wurden in 416 g Toluol bei 60 °C in einem 2 L Autoklaven vorgelegt und mit Stickstoff geflutet. Die Temperatur wurde anschließend auf 120 °C erhöht. 1057 g (24,0 mol) Ethylenoxid wurden innerhalb von 13 h zugegeben. Das erhaltene Reaktionsgemisch wurde 10 h bei 120 °C nachgerührt und nach Abkühlen auf 105 °C am Vakuum von flüchtigen Bestandteilen befreit.
Es wurden 1181 g eines hellbraunen Feststoffes erhalten.
OHZ = 50,6 mg KOH/g (Theorie 48,8 mg KOH/g), PEG-Gehalt: < 1 Gew%

### Beispiel 1e:

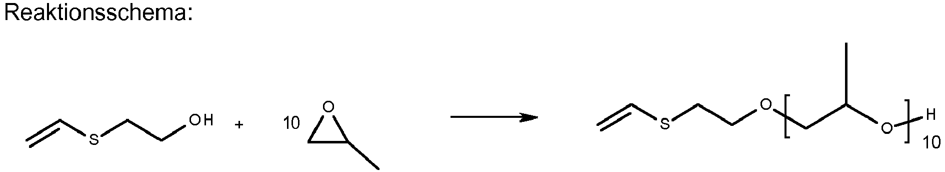

104 g (1,00 mol) Vinylmercaptoethanol und 641 mg (2,97 mmol KOMe) einer methanolischen Kaliummethylatlösung (32 gew.-%ig in Methanol) wurden bei 60 °C in einem 2 L Autoklaven vorgelegt und das Methanol bei <20 mbar innerhalb 2 h entfernt. Der Autoklav wurde anschließend mit Stickstoff geflutet und die Temperatur auf 130 °C erhöht. 581 g (10,0 mol) Propylenoxid wurden innerhalb von 10 h zugegeben. Das erhaltene Reaktionsgemisch wurde 10 h bei 130 °C nachgerührt und nach Abkühlen auf 100 °C am Vakuum von flüchtigen Bestandteilen befreit.
Es wurden 629 g eines dunkelbraunen Feststoffes erhalten.
OHZ = 88,0 mg KOH/g (Theorie 81,9 mg KOH/g)

### Beispiel 1f:

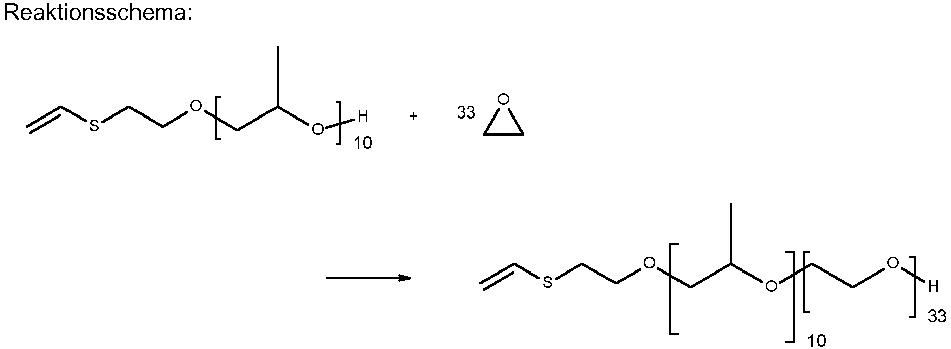

188 g (275 mmol) Vinylmercaptoethanol*10 PO (Beispiel 1e) und 1,85 g (16,6 mmol KOH) einer wässrigen Kaliumhydroxidlösung (50 Gew.-%ig) wurden bei 100 °C in einem 2 L Autoklaven vorgelegt und bei <20 mbar innerhalb 2 h entwässert. Der Autoklav wurde anschließend mit Stickstoff geflutet und die Temperatur auf 120 °C erhöht. 396 g (9,00 mol) Ethylenoxid wurden innerhalb von 6 h zugegeben. Das erhaltene Reaktionsgemisch wurde 5 h bei 120 °C nachgerührt und nach Abkühlen auf 90 °C am Vakuum von flüchtigen Bestandteilen befreit.
Es wurden 597 g eines hellbraunen Feststoffes erhalten.
OHZ= 31,8 mg KOH/g (Theorie 28,8).

### Beispiel 1g:

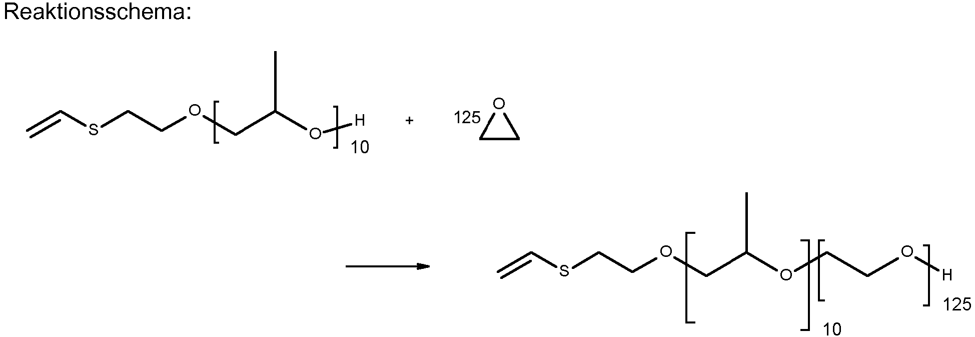

68,4 g (0,10 mmol) Vinylmercaptoethanol*10 PO (Beispiel 1e) und 0,62 g (5,52 mmol KOH) einer wässrigen Kaliumhydroxidlösung (50 Gew.-%ig) wurden bei 100 °C in einem 2 L Autoklaven vorgelegt und bei <20 mbar innerhalb 2 h entwässert. Der Autoklav wurde anschließend mit Stickstoff geflutet und die Temperatur auf 120 °C erhöht. 550 g (12,5 mol) Ethylenoxid wurden innerhalb von 6 h zugegeben. Das erhaltene Reaktionsgemisch wurde 5 h bei 120 °C nachgerührt und nach Abkühlen auf 90 °C am Vakuum von flüchtigen Bestandteilen befreit.
Es wurden 610 g eines braunen Feststoffes erhalten.
OHZ= 12,2 mg KOH/g (Theorie 9,2), PEG-Gehalt: 1,4 Gew%

### Beispiel 1h:

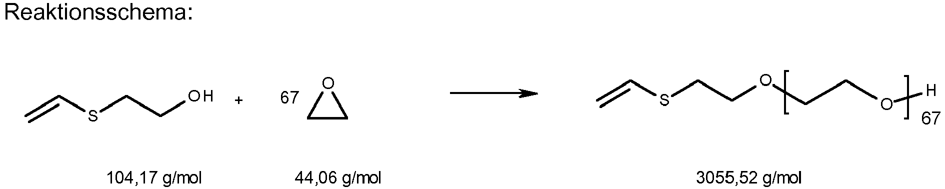

46,7 g (448 mmol) Vinylmercaptoethanol und 120 mg (1,71 mmol) Kaliummethanolat wurden in 100 ml Toluol bei 60 °C in einem 2 L Autoklaven vorgelegt und mit Stickstoff geflutet. Die Temperatur wurde anschließend auf 120 °C erhöht. 1321 g (30,0 mol) Ethylenoxid wurden innerhalb von 20 h zugegeben. Das erhaltene Reaktionsgemisch wurde 10 h bei 120 °C nachgerührt und nach Abkühlen auf 100 °C am Vakuum von flüchtigen Bestandteilen befreit.
Es wurden 1396 g eines hellbraunen Feststoffes erhalten.
OHZ = 21,2 mg KOH/g (Theorie 18,4 mg KOH/g), PEG-Gehalt: 1,4 Gew%

### Beispiel 1i:

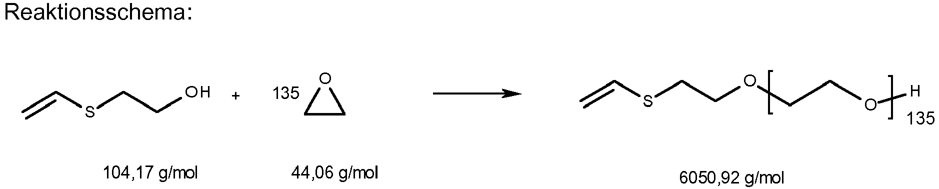

23,4 g (224 mmol) Vinylmercaptoethanol und 120 mg (1,71 mmol) Kaliummethanolat wurden in 50 ml Toluol bei 60 °C in einem 2 L Autoklaven vorgelegt und mit Stickstoff geflutet. Die Temperatur wurde anschließend auf 120 °C erhöht. 1332 g (30,2 mol) Ethylenoxid wurden innerhalb von 36 h zugegeben. Das erhaltene Reaktionsgemisch wurde 12 h bei 120 °C nachgerührt und nach Abkühlen auf 100 °C am Vakuum von flüchtigen Bestandteilen befreit.
Es wurden 1388 g eines hellbraunen Feststoffes erhalten.
OHZ = 12,1 mg KOH/g (Theorie 9,3 mg KOH/g), PEG-Gehalt: 2,0 Gew-%

### Vergleichsbeispiel 1j:

Herstellung von HBVE- 24 EO

Es wurde wie in Beispiel 1 c vorgegangen, nur wurde anstelle von VME eine entsprechende Menge HBVE eingesetzt.

### Vergleichsbeispiel 1k:

Herstellung von HBVE- 67 EO

Es wurde wie in Beispiel 1 h vorgegangen, nur wurde anstelle von VME eine entsprechende Menge HBVE eingesetzt.

### Vergleichsbeispiel 11:

Herstellung von HBVE- 135 EO

Es wurde wie in Beispiel 1 i vorgegangen, nur wurde anstelle von VME eine entsprechende Menge HBVE eingesetzt.

### Beispiel 2: Herstellung von Polymeren, Verfahren (A)

Beim Verfahren (A) werden die VME- bzw. HBVE-Alkoxylate zur Polymerisation vorgelegt

### Beispiel 2a:

Copolymer aus 89 Gew.-% VME-23EO und 11 Gew.-% Acrylsäure
Polymerisation bei pH 2-3 (Azostarter)

In einem mit Rührer, Rückflußkühler, Thermometer, Stickstoffleitung und Dosierungsleitungen ausgestatteten Glasreaktor wurden 35,6 g VME-Ethoxylat (23EO) gemäß Beispiel 1b in 63,5 g Wasser aufgelöst. Eine Teilmenge (1,1 g) einer Lösung A (4,4 g Acrylsäure gelöst in 18,4g Wasser) und eine Teilmenge (2 g) einer Lösung B (1,6 g 2,2'-Azobis[2-(2-imidazolin-2-yl)propan]dihydrochlorid (Wako VA-44) gelöst in 38,1 g Wasser) wurden zugegeben. Die Polymerisation wurde durch Erhitzen der Lösung unter Stickstoff auf 70°C gestartet, und anschließend wurden die Restmengen der Lösungen A und B zugegeben, wobei die Lösung A in 3 Stunden und die Lösung B in 4 Stunden zugegeben wurde. Anschließend wurde das Reaktionsmischung 2 Stunden bei 70 °C gerührt.
Daten zur Synthese sowie Mₙ, M_{w} und PDI des erhaltenen Polymers sind in Tabelle 1 zusammengefasst.

### Beispiel 2b:

Es wurde wie in Beispiel 2a vorgegangen, nur wurde die Menge des Initiators von 1,6 g auf 1,2 g reduziert. Daten zur Synthese sowie Mₙ, M_{w} und PDI des erhaltenen Polymers sind in Tabelle 1 zusammengefasst.

### Beispiel 2c:

Es wurde wie in Beispiel 2a vorgegangen, nur wurde die Menge des Initiators von 1,6 g auf 0,8 g reduziert. Daten zur Synthese sowie Mₙ, M_{w} und PDI des erhaltenen Polymers sind in Tabelle 1 zusammengefasst.

### Vergleichsbeispiel 2a:

Copolymer aus 89 Gew.-% HBVE-24EO und 11 Gew.-% Acrylsäure

In einem mit Rührer, Rückflußkühler, Thermometer, Stickstoffleitung und Dosierungsleitungen ausgestatteten Glasreaktor wurden 35,6 g HBVE-24EO gemäß Vergleichsbeispiel 1i in 63,5 g Wasser aufgelöst. Eine Teilmenge (1,1g) einer Lösung A (4,4 g Acrylsäure gelöst in 18,4 g Wasser) und eine Teilmenge (2 g) einer Lösung B (1,6 g 2,2'-Azobis[2-(2-imidazolin-2-yl)propane]dihydrochlorid (Wako VA-44) gelöst in 38,1g Wasser) wurden zugegeben. Die Polymerisation wurde durch Erhitzen der Lösung unter Stickstoff auf 70°C gestartet, und anschließend wurden die Restmengen der Lösungen A und B zugegeben, wobei die Lösung A in 3 Stunden und die Lösung B in 4 Stunden zugegeben wurde. Anschließend wurde das Reaktionsmischung 2 Stunden bei 70 °C gerührt.
Daten zur Synthese sowie Mₙ, M_{w} und PDI des erhaltenen Polymers sind in Tabelle 1 zusammengefasst.

### Vergleichsbeispiel 2b:

Es wurde wie in Beispiel 2 vorgegangen, nur wurde die Menge des Initiators von 1,6 g auf 1,2 g reduziert. Daten zur Synthese sowie Mₙ, M_{w} und PDI des erhaltenen Polymers sind in Tabelle 1 zusammengefasst.

### Vergleichsbeispiel 2c:

Es wurde wie in Beispiel 2 vorgegangen, nur wurde die Menge des Initiators von 1,6 g auf 0,8 g reduziert. Daten zur Synthese sowie Mₙ, M_{w} und PDI des erhaltenen Polymers sind in Tabelle 1 zusammengefasst.

Eine GPC-Messung der Reaktionsprodukte aus Beispiel 2a und Vergleichsbeispiel 2a zeigte einen zusätzlichen stark ausgeprägten niedermolekularen Peak im Fall des HBVE-24EO (Vergleichsversuch 2a). Im Falle der HBVE-Ethoxylate wurde ein großer Anteil der Monomere während der Polymerisation hydrolysiert.

Weiterhin wurde in 1H-NMR Messungen der Vergleichsbeispiele 2a, 2b und 2c keine Vinylverbindung mehr beobachtet.

### Beispiel 2 d:

Copolymer aus 85 Gew.-% VME-23EO und 15 Gew.-% Na-Acrylat
Polymerisation bei pH 7-8 (Azostarter)

In einem mit Rührer, Rückflußkühler, Thermometer, Stickstoffleitung und Dosierungsleitungen ausgestatteten Glasreaktor wurden 34,0 g VME-Ethoxylat-23EO gemäß Beispiel 1b in 63,5g Wasser aufgelöst. Eine Teilmenge (1,2 g) einer Lösung A (6,0 g Acrylsäure -Na-Salz gelöst in 18,4g Wasser) und eine Teilmenge (2 g) einer Lösung B (1,6 g 2,2'-Azobis[2-(2-imidazolin-2-yl)propane] dihydrochlorid (Wako VA-44) gelöst in 38,1 g Wasser) wurden zugegeben. Die Polymerisation wurde durch Erhitzen der Lösung unter Stickstoff auf 70°C gestartet, und anschließend wurden die Restmengen der Lösungen A und B zugegeben, wobei die Lösung A in 3 Stunden und die Lösung B in 4 Stunden zugegeben wurde. Anschließend wird das Reaktionsmischung 2 Stunden bei 70 °C gerührt.
Daten zur Synthese sowie Mₙ, M_{w} und PDI des erhaltenen Polymers sind in Tabelle 1 zusammengefasst.

### Vergleichsbeispiel 2d:

Copolymer aus 85 Gew.-% HBVE-24EO und 15 Gew.-% Na-Acrylat
Polymerisation bei pH 7-8 (Azostarter)

In einem mit Rührer, Rückflußkühler, Thermometer, Stickstoffleitung und Dosierungsleitungen ausgestatteten Glasreaktor wurden 34,0 g HBVE-24EO gemäß Beispiel 1i in 63,5 g Wasser aufgelöst. Eine Teilmenge (1,2g) einer Lösung A (6,0g Acrylsäure-Na-Salz gelöst in 18,4g Wasser) und eine Teilmenge (2g) einer Lösung B (1,6g 2,2'-Azobis[2-(2-imidazolin-2-yl)propane]dihydrochlorid (Wako VA-44) gelöst in 38,1g Wasser) wurden zugegeben. Die Polymerisation wurde durch Erhitzen der Lösung unter Stickstoff auf 70°C gestartet, und anschließend wurden die Restmengen der Lösungen A und B zugegeben, wobei die Lösung A in 3 Stunden und die Lösung B in 4 Stunden zugegeben wurde. Anschließend wurde das Reaktionsmischung 2 Stunden bei 70 °C gerührt.

Bei neutralem pH wurde keine Hydrolyse von HBVE-Ethoxylat beobachtet. ¹H-NMR Messung des Polymerisats ergeben, dass ca. 8% des eingesetzten HBVE-Ethoxylats nicht polymerisiert waren. Die Molmasse des HBVE-Ethoxylat/Acrylsäure Copolymers war deutlich niedriger als die des VME-Ethoxylat-Acrylsäure Copolymers.
Daten zur Synthese sowie Mₙ, M_{w} und PDI des erhaltenen Polymers sind in Tabelle 1 zusammengefasst.

### Beispiel 2e:

Copolymer aus 89 Gew.-% VME-23EO und 11 Gew.-% Acrylsäure
Polymerisation bei pH 1,9 (Peroxidstarter)

In einem mit Rührer, Rückflußkühler, Thermometer, Stickstoffleitung und Dosierungsleitungen ausgestatteten Glasreaktor wurden 42,7g VME-Ethoxylat-24EO gemäßBeispiel 1d in 38,8 g Wasser aufgelöst. Die Lösung wurde unter Stickstoff auf 95°C aufgeheizt und Zugabe von Lösungen A (5,3 g Acrylsäure gelöst in 41,4g Wasser) und B (1,9 g Natriumperoxodisulfat gelöst in 31,4g Wasser) gestartet, wobei die Lösung A 4 Stunden und die Lösung B in 5 Stunden zugegeben wurden. Anschließend wurde das Reaktionsmischung 2 Stunden bei 95 °C gerührt. Daten zur Synthese sowie Mₙ, M_{w} und PDI des erhaltenen Polymers sind in Tabelle 1 zusammengefasst.

### Vergleichsbeispiel 2e:

Copolymer aus 89 Gew.-% HBVE-24EO und 11 Gew.-% Acrylsäure
Polymerisation bei pH 1,9

In einem mit Rührer, Rückflußkühler, Thermometer, Stickstoffleitung und Dosierungsleitungen ausgestatteten Glasreaktor wurden 42,7g HBVE-24EO gemäß Vergleichsbeispiel 1j in 38,8g Wasser aufgelöst. Die Lösung wurde unter Stickstoff auf 95°C aufgeheizt und Zugabe von Lösungen A (5,3g Acrylsäure gelöst in 41,4g Wasser) und B (1,9g Natriumperoxodisulfat gelöst in 31,4g Wasser) gestartet, wobei die Lösung A4 Stunden und die Lösung B in 5 Stunden zugegeben wurden. Anschließend wurde das Reaktionsmischung 2 Stunden bei 95 °C gerührt.
Es wurde eine starke Hydrolyse von HBVE-Ethoxylat beobachtet. Im GPC wurde ein großer Peak bei kleinen Molmassen beobachtet.
Daten zur Synthese sowie Mₙ, M_{w} und PDI des erhaltenen Polymers sind in Tabelle 1 zusammengefasst.

### Beispiel 2f: Polymerisation in Masse

Copolymer aus 89 Gew.-% VME-24EO und 11 Gew.-% Acrylsäure

In einem mit Rührer, Rückflußkühler, Thermometer, Stickstoffleitung und Dosierungsleitungen ausgestatteten Glasreaktor wurden 71,2g VME-24EO gemäß Beispiel 1d und 2,4g 2-Mercaptoethanol vorgelegt. Die Mischung wurde unter Stickstoff auf 85°C aufgeheizt und Zugabe 8,7 g Acrylsäure und 3,2g tert-Butylperoctoat gestartet, wobei die Acrylsäure in 3 Stunden und Initiator in 4 Stunden zugegeben wurden. Anschließend wurde das Reaktionsmischung 4 Stunden bei 85 °C gerührt.
Daten zur Synthese sowie Mₙ, M_{w} und PDI des erhaltenen Polymers sind in Tabelle 1 zusammengefasst.

### Vergleichsbeispiel 2f:

Copolymer aus 89 Gew.-% HBVE-24EO und 11 Gew.-% Acrylsäure

In einem mit Rührer, Rückflußkühler, Thermometer, Stickstoffleitung und Dosierungsleitungen ausgestatteten Glasreaktor wurden 71,2 g HBVE-24EO gemäß Vergleichsbeispiel 1j und 2,4g 2-Mercaptoethanol vorgelegt .Die Mischung wurde unter Stickstoff auf 85°C aufgeheizt und Zugabe 8,7 g Acrylsäure und 3,2g tert-Butylperoctoat gestartet, wobei die Acrylsäure in 3 Stunden und Initiator in 4 Stunden zugegeben wurden. Anschließend wurde das Reaktionsmischung 4 Stunden bei 85 °C gerührt.
Daten zur Synthese sowie Mₙ, M_{w} und PDI des erhaltenen Polymers sind in Tabelle 1 zusammengefasst.

Erfindungsgemäße Polymere lassen sich auch in Masse (ohne Lösungsmittel) herstellen. Zur Polymerisation in Masse werden die Monomere, mit einem Azo-Starter versetzt. Beispielsweise ist auch die Copolymerisation von VME Ethoxylat und Acrylsäure in Masse mit Peroctoat als Initiator bei 85°C möglich. Bei der Polymerisation in Masse können auch Regler, beispielsweise Mercaptoethanol oder Mercaptopropionsäure eingesetzt werden.

### Beispiel 2g: Polymerisation von langkettigen VME-Ethoxylaten

Copolymer aus 81 Gew.-% VME-67EO und 19 Gew.-% Acrylsäure
Polymerisation bei pH 2-3 (Azostarter)

In einem mit Rührer, Rückflußkühler, Thermometer, Stickstoffleitung und Dosierungsleitungen ausgestatteten Glasreaktor wurden 32,6 g VME-Ethoxylat (67EO) gemäß Beispiel 1h in 63,5 g Wasser aufgelöst. Eine Teilmenge (1,3 g) einer Lösung A (7,6 g Acrylsäure gelöst in 18,4g Wasser) und eine Teilmenge (2 g) einer Lösung B (1,6 g 2,2'-Azobis[2-(2-imidazolin-2-yl)propane]dihydrochlorid (Wako VA-44) gelöst in 38,1 g Wasser) wurden zugegeben. Die Polymerisation wurde durch Erhitzen der Lösung unter Stickstoff auf 70°C gestartet, und anschließend wurden die Restmengen der Lösungen A und B zugegeben, wobei die Lösung A in 3 Stunden und die Lösung B in 4 Stunden zugegeben wurde. Anschließend wurde das Reaktionsmischung 2 Stunden bei 70 °C gerührt.
Daten zur Synthese sowie Mₙ, M_{w} und PDI des erhaltenen Polymers sind in Tabelle 1 zusammengefasst.

### Vergleichsbeispiel 2g:

Copolymer aus 81 Gew.-% HBVE-67EO und 19 Gew.-% Acrylsäure
Polymerisation bei pH 2-3 (Azostarter)

In einem mit Rührer, Rückflußkühler, Thermometer, Stickstoffleitung und Dosierungsleitungen ausgestatteten Glasreaktor wurden 32,4 g HBVE-Ethoxylat (67EO) gemäß Vergleichsbeispiel 1k in 57,6g Wasser aufgelöst. Eine Teilmenge (1,6 g) einer Lösung A (7,6 g Acrylsäure gelöst in 24,0g Wasser) und eine Teilmenge (2 g) einer Lösung B (1,6 g 2,2'-Azobis[2-(2-imidazolin-2-yl)propane]dihydrochlorid (Wako VA-44) gelöst in 38,4g Wasser) wurden zugegeben. Die Polymerisation wurde durch Erhitzen der Lösung unter Stickstoff auf 70°C gestartet, und anschließend wurden die Restmengen der Lösungen A und B zugegeben, wobei die Lösung A in 3 Stunden und die Lösung B in 4 Stunden zugegeben wurde. Anschließend wurde das Reaktionsmischung 2 Stunden bei 70 °C gerührt.
Daten zur Synthese sowie Mₙ, M_{w} und PDI des erhaltenen Polymers sind in Tabelle 1 zusammengefasst.

Eine GPC-Messung der Reaktionsprodukte aus Beispiel 2g und Vergleichsbeispiel 2g zeigte einen zusätzlichen stark ausgeprägten niedermolekularen Peak im Fall des HBVE-24EO (Vergleichsversuch 2g). Im Falle der HBVE-Ethoxylate wurde ein großer Anteil der Monomere während der Polymerisation hydrolysiert. Weiterhin wurde in 1H-NMR Messungen der Vergleichsbeispiele 2g Vinylverbindung mehr beobachtet.

### Beispiel 2h:

Copolymer aus 81 Gew.-% VME-135EO und 19 Gew.-% Acrylsäure
Polymerisation bei pH 2-3 (Azostarter)

In einem mit Rührer, Rückflußkühler, Thermometer, Stickstoffleitung und Dosierungsleitungen ausgestatteten Glasreaktor wurden 32,3 g VME-Ethoxylat (135EO) in 63,5 g Wasser aufgelöst. Eine Teilmenge (1,3 g) einer Lösung A (7,7g Acrylsäure gelöst in 18,4g Wasser) und eine Teilmenge (2 g) einer Lösung B (1,6 g 2,2'-Azobis[2-(2-imidazolin-2-yl)propane]dihydrochlorid (Wako VA-44) gelöst in 38,2g Wasser) wurden zugegeben. Die Polymerisation wurde durch Erhitzen der Lösung unter Stickstoff auf 70°C gestartet, und anschließend wurden die Restmengen der Lösungen A und B zugegeben, wobei die Lösung A in 3 Stunden und die Lösung B in 4 Stunden zugegeben wurde. Anschließend wurde das Reaktionsmischung 2 Stunden bei 70 °C gerührt.
Daten zur Synthese sowie Mₙ, M_{w} und PDI des erhaltenen Polymers sind in Tabelle 1 zusammengefasst.

### Vergleichsbeispiel 2h:

Copolymer aus 81 Gew.-% HBVE-135EO und 19 Gew.-% Acrylsäure
Polymerisation bei pH 2-3 (Azostarter)

In einem mit Rührer, Rückflußkühler, Thermometer, Stickstoffleitung und Dosierungsleitungen ausgestatteten Glasreaktor wurden 32,3g HBVE-Ethoxylat (135EO) gemäß Vergleichsbeispiel 1 l in 57,6 g Wasser aufgelöst. Eine Teilmenge (1,6 g) einer Lösung A (7,7 g Acrylsäure gelöst in 24,0g Wasser) und eine Teilmenge (2 g) einer Lösung B (1,6 g 2,2'-Azobis[2-(2-imidazolin-2-yl)propane]dihydrochlorid (Wako VA-44) gelöst in 38,4g Wasser) wurden zugegeben. Die Polymerisation wurde durch Erhitzen der Lösung unter Stickstoff auf 70°C gestartet, und anschließend wurden die Restmengen der Lösungen A und B zugegeben, wobei die Lösung A in 3 Stunden und die Lösung B in 4 Stunden zugegeben wurde. Anschließend wurde das Reaktionsmischung 2 Stunden bei 70 °C gerührt.
Daten zur Synthese sowie Mₙ, M_{w} und PDI des erhaltenen Polymers sind in Tabelle 1 zusammengefasst.

**Tabelle 1: Ergebnisse der Polymersynthesen (*Restmonomermenge mittels NMR, AS = Acrylsäure, Mengengaben Gew.-%)**

| **Versuch** | **Monomere** | **Initiator** | | **pH während Polymerisation** | **Mₙ [g/mol** | **M_{w} (g/mol)** | **M_{w}/Mₙ** | **Menge Restmonomer [Mol-%]*** |
|---|---|---|---|---|---|---|---|---|
| | | **Art** | **Menge** | | | | | |
| Beispiel 2a | 89 % VME-23EO , 11 % AS | Azo | 1,6 g | 2,4 | 6348 | 34566 | 5,44 | |
| Beispiel 2b | 89 % VME-23EO , 11 % AS | Azo | 1,2 g | 2,3 | 6137 | 41648 | 6,78 | |
| Beispiel 2c | 89 % VME-23EO , 11 % AS | Azo | 0,8 g | 2,4 | 6591 | 42874 | 6,50 | |
| Vergleichsbeispiel 2a | 89 % HBVE-24EO, 11% AS | Azo | 1,6g | 2,4 | 1267 | 12459 | 9,83 | |
| Vergleichsbeispiel 2b | 89 % HBVE-24EO, 11% AS | Azo | 1,2 g | 2,4 | 1141 | 4366 | 3,82 | |
| Vergleichsbeispiel 2c | 89 %HBVE-24EO, 11 % AS | Azo | 0,8 g | 2,5 | 1478 | 17994 | 12,20 | |
| Beispiel 2d | 85 % VME-23 EO, 11 % AS | Azo | 1,6g | 8,1 | 4974 | 20834 | 4,18 | 0,0 |
| Vergleichsbeispiel 2d | 85 % HBVE-24EO, 11 % AS | Azo | 1,6 g | 7,8 | 1928 | 9555 | 4,95 | 8,2 |
| Beispiel 2e | 89 % VME-24 EO, 11 % AS | Peroxid | 1,9 g | 1,9 | 2629 | 7008 | 2,66 | |
| Vergleichsbeispiel 2e | 89 % HBVE-24 EO, 11% AS | Peroxid | 1,9g | 1,9 | 864 | 2701 | 3,12 | |
| Beispiel 2f | 89 % VME-24 EO, 11 % AS | Peroxid | 3,2 g | - | 14637 | 139210 | 9,51 | n.b. |
| Vergleichsbeispiel 2f | 89 % HBVE-24 EO, 11% AS | Peroxid | 3,2 g | - | 7727 | 13592 | 1,75 | n.b |
| Beispiel 2g | 81 % VME-67 EO, 19 % AS | Azo | 1,6g | 2,5 | 28707 | 140920 | 4,90 | |
| Vergleichsbeispiel 2g | 81 % HBVE-67 EO, 19% AS | Azo | 1,6g | 2,4 | 8605 | 37977 | 4,41 | |
| Beispiel 2h | 81 % VME-135 EO, 19 % AS | Azo | 1,6 g | 2,5 | 23495 | 159210 | 6,78 | |
| Vergleichsbeispiel 2h | 81 % HBVE-135 EO, 19% AS | Azo | 1,6g | 2,4 | 14976 | 56306 | 3,76 | |

### Beispiel 3: Herstellung von Polymeren, (Verfahren (B)

Beim Verfahren (B) werden die VME- bzw. HBVE-Alkoxylate zur Polymerisation nicht vorgelegt, sondern während der Polymerisation nach und nach zugegeben.

### Beispiel 3a:

Copolymer aus 89 Gew.-% VME-23EO und 11 Gew.-% Acrylsäure (Molverhältnis 1:2)
Polymerisation bei pH 2,8 (Peroxidstarter)

In einem mit Rührer, Rückflußkühler, Thermometer, Stickstoffleitung und Dosierungsleitungen ausgestatteten Glasreaktor wurde 66 g Wasser, eine Teilmenge (2,9 g) von Lösung A (35,6 g VME-23EO gemäß Beispiel 1b sowie 4,4 g Acrylsäure gelöst in 18 g Wasser) und eine Teilmenge (1,9 g) von Lösung B (0,8 g (75% Lösung) tert-Butylperpivalat und 36 g iso-Propanol) vorgelegt. Die Vorlage wurde unter Stickstoff auf 75°C aufgeheizt und die Polymerisation Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 75 °C gerührt. Iso-Propanol wurde abdestilliert.
Daten zur Synthese sowie Mₙ, M_{w} und PDI des erhaltenen Polymers sind in Tabelle 2 zusammengefasst.

### Beispiel 3b:

Copolymer aus 89 Gew.-% VME-23EO und 11 Gew.-% Acrylsäure
Polymerisation bei pH 2,6 (Azo-Starter)

In einem mit Rührer, Rückflußkühler, Thermometer, Stickstoffleitung und Dosierungsleitungen ausgestatteten Glasreaktor wurde 66 g Wasser, eine Teilmenge (2,9 g) von Lösung A (35,6 g VME-23EO gemäß Beispiel 1b, 4,4 g Acrylsäure gelöst in 18g Wasser) und eine Teilmenge (1,9 g) von Lösung B (1,6 2,2'-Azobis[2-(2-imidazolin-2-yl)propan]dihydrochlorid (Wako VA-44) und 36 g Wasser) vorgelegt. Die Vorlage wurde unter Stickstoff auf 70°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 70 °C gerührt.
Daten zur Synthese sowie Mₙ, M_{w} und PDI des erhaltenen Polymers sind in Tabelle 2 zusammengefasst.

### Vergleichsbeispiel 3a:

Copolymer aus 86 Gew.-% HBVE-24 EO und 14 Gew.-% Na-Acrylat
Polymerisation bei pH 8,5 (Peroxidstarter)

In einem mit Rührer, Rückflußkühler, Thermometer, Stickstoffleitung und Dosierungsleitungen ausgestatteten Glasreaktor wurde 288,7 g Wasser, eine Teilmenge (14 g) von Lösung A (150,8 g HBVE-24EO gemäß Beispiel 1i, 80,6 g 30%-ge Acrylsäure Na-Salz Lösung und 48,5 g Wasser) und eine Teilmenge (6,7 g) von Lösung B (3,5 g (75% Lösung) tert-Betylperpivalat und 131,2 g iso-Propanol gelöst) vorgelegt. Die Vorlage wurde unter Stickstoff auf 75°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 75 °C gerührt. Die Reaktion Mischung wurde konstant bei pH 7,5-8,0 gehalten. Anschließend wurde iso-Propanol abdestilliert.
Daten zur Synthese sowie Mₙ, M_{w} und PDI des erhaltenen Polymers sind in Tabelle 2 zusammengefasst.

### Vergleichsbeispiel 3b

Copolymer aus 86 Gew.-% HBVE-24 EO und 14 Gew.-% Na-Acrylat
Polymerisation bei pH 2-3 (Azo-Starter)

In einem mit Rührer, Rückflußkühler, Thermometer, Stickstoffleitung und Dosierungsleitungen ausgestatteten Glasreaktor wurde 252 g Wasser, eine Teilmenge (14 g) von Lösung A (150,8 g HBVE-24EO gemäß Beispiel 1i, 80,6 g 30%-ge Acrylsäure Na-Salz Lösung und 48,5 g Wasser) und eine Teilmenge (8,6 g) von Lösung B (3,5 g 2,2'-Azobis[2-(2-imidazolin-2-yl)propane]dihydrochlorid (Wako VA-44) und 168 g Wasser) vorgelegt. Die Vorlage wurde unter Stickstoff auf 70°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 70 °C gerührt. Die Reaktion Mischung wurde konstant bei pH 7,5-8,0 gehalten.
Daten zur Synthese sowie Mₙ, M_{w} und PDI des erhaltenen Polymers sind in Tabelle 2 zusammengefasst.

### Beispiel 3c:

Copolymer aus 97 Gew.-% VME-135EO und 3 Gew.-% Na-Acrylat
Polymerisation bei pH 9,4 (Peroxidstarter)

In einem mit Rührer, Rückflußkühler, Thermometer, Stickstoffleitung und Dosierungsleitungen ausgestatteten Glasreaktor wurde 39,4 g Wasser, eine Teilmenge (4,4 g) von Lösung A (36,4 g VME-135EO gemäß Beispiel 1h, 3,7 g 30%-ge Acrylsäure Na-Salz Lösung und 48g Wasser) und eine Teilmenge (1,16 g) von Lösung B (0,75 g (75% Lösung) tert-Betylperpivalat und 22 g iso-Propanol gelöst) vorgelegt. Die Vorlage wurde unter Stickstoff auf 75°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 75 °C gerührt. Iso-Propanol wurde abdestilliert.
Daten zur Synthese sowie Mₙ, M_{w} und PDI des erhaltenen Polymers sind in Tabelle 2 zusammengefasst.

### Beispiel 3d:

Copolymer aus 97 Gew.-% VME-135EO und 3 Gew.-% Na-Acrylat
Polymerisation bei pH 8,6 (Azo-Starter)

In einem mit Rührer, Rückflußkühler, Thermometer, Stickstoffleitung und Dosierungsleitungen ausgestatteten Glasreaktor wurde 45 g Wasser, eine Teilmenge (4,4 g) von Lösung A (36,7g VME-135EO gemäß Beispiel 1h, 3,7 g 30%-ge Acrylsäure Na-Salz Lösung und 48g Wasser) und eine Teilmenge (0,88 g) von Lösung B (0,75 g 2,2'-Azobis[2-(2-imidazolin-2-yl)propane]dihydrochlorid (Wako VA-44) und 17 g Wasser) vorgelegt. Die Vorlage wurde unter Stickstoff auf 70°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 70 °C gerührt.
Daten zur Synthese sowie Mₙ, M_{w} und PDI des erhaltenen Polymers sind in Tabelle 2 zusammengefasst.

### Vergleichsbeispiel 3c:

Copolymer aus 94 Gew.-% HBVE-135EO und 6 Gew.-% Na-Acrylat
Polymerisation bei pH 7,7 (Peroxidstarter)

In einem mit Rührer, Rückflußkühler, Thermometer, Stickstoffleitung und Dosierungsleitungen ausgestatteten Glasreaktor wurde 238,7 g Wasser, eine Teilmenge (14,0 g) von Lösung A (164,9 g HBVE-135 EO gemäß Vergleichsbeispiel 1j, 33,7 g 30%-ge Acrylsäure Na-Salz Lösung und 131,4 g Wasser) und eine Teilmenge (6,7 g) von Lösung B (3,5 g (75% Lösung) tert-Betylperpivalat und 131,2 g iso-Propanol gelöst) vorgelegt. Die Vorlage wurde unter Stickstoff auf 75°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 75 °C gerührt. Die Reaktion Mischung wurde konstant bei pH 7,5-8,0 gehalten. Anschließend wurde iso-Propanol abdestilliert.
Daten zur Synthese sowie Mₙ, M_{w} und PDI des erhaltenen Polymers sind in Tabelle 2 zusammengefasst.

**Tabelle 2: Ergebnisse der Polymersynthesen (*Restmonomermenge mittels NMR, AS = Acrylsäure bzw. Na-Acrylat, Mengengaben Gew.-%)**

| **Versuch** | **Monomere** | **Initiator** | | **pH während Polymerisation** | **Mₙ [g/mol** | **Mw (g/mol)** | **Mw/Mn** | **Nicht umgesetztes Ethoxylatmonomer [%]*** |
|---|---|---|---|---|---|---|---|---|
| | | **Art** | **Menge** | | | | | |
| Beispiel 3a | 89 % VME-23EO, 11 % AS | Peroxid | 1,9 g | 2,8 | 27709 | 42697 | 1,54 | 0,0 |
| Vergleichsbeispiel 3a | 86 % HBVE-24EO, 14 % AS | Peroxid | 3,5 g | 8,7 | 12238 | 15404 | 1,26 | 17 |
| Beispiel 3b | 89 % VME-23EO, 11 % AS | Azo | 1,9g | 2,6 | 23197 | 37150 | 1,6 | 0,0 |
| Vergleichsbeispiel 3b | 86 % HBVE-24EO, 14 % AS | Azo | 8,6 g | 8,5 | 18028 | 25349 | 1,41 | 50 |
| Beispiel 3c | 97 % VME-135EO, 3 % AS | Peroxid | 0,75 g | 9,4 | 15682 | 20930 | 1,33 | 0,0 |
| Vergleichsbeispiel 3c | 94 % HBVE-135EO, 6 % AS | Peroxid | 3,5 g | 7,7 | 36819 | 39428 | 1,07 | 37 |
| Beispiel 3d | 97 % VME-135EO, 3 % AS | Azo | 0,75 g | 8,6 | 18776 | 29705 | 1,58 | 0 |

### Beispiel 4:

### Polymerisation von VME-Ethoxylaten mit Methacrylsäure

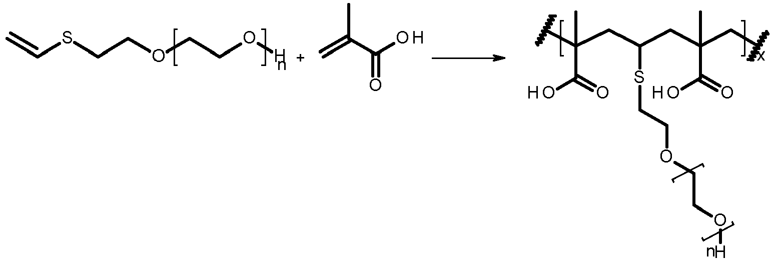

### Beispiel 4a:

Copolymer aus 87 Gew.-% VME-24EO und 13 Gew.-% Na-Methacrylat
Polymerisation bei pH 7,5-8,0 (Azo-Starter), Verfahren (A)

In einem mit Rührer, Rückflusskühler, Thermometer, Stickstoffleitung und Dosierungsleitungen ausgestatteten Glasreaktor wurde 57 g Wasser, 34,8 g VME-24EO eine Teilmenge (1,4 g) von Lösung A (17,2 g 30%-ge Methacrylsäure Na-Salz Lösung und 12g Wasser) und eine Teilmenge (2,0 g) von Lösung B (1,6 g 2,2'-Azobis[2-(2-imidazolin-2-yl)propane]dihydrochlorid (Wako VA-44) und 38,4 g Wasser) vorgelegt. Die Vorlage wurde unter Stickstoff auf 70°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 70 °C gerührt.
Die Ergebnisse sind in Tabelle 3 zusammengefasst.

### Beispiel 4b:

Copolymer aus 82 Gew.-% VME-23EO und 18 Gew.-% Na-methacrylat
Polymerisation bei pH 7,5-8,0 (Peroxidstarter), Verfahren (A)

In einem mit Rührer, Rückflusskühler, Thermometer, Stickstoffleitung und Dosierungsleitungen ausgestatteten Glasreaktor wurde 70 g Wasser, 33 g VME-23EO, eine Teilmenge (2,1 g) von Lösung A (24 g 30%-ge Methacrylsäure Na-Salz Lösung und 20g Wasser) und eine Teilmenge (0,8 g) von Lösung B (1,6 g (75% Lösung) tert-Betylperpivalat und 14,4 g iso-Propanol) vorgelegt. Die Vorlage wurde unter Stickstoff auf 75°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 75 °C gerührt. Iso-Propanol wurde abdestilliert.
Die Ergebnisse sind in Tabelle 3 zusammengefasst.

### Beispiel 4c:

Copolymer aus 87 Gew.-% VME-23EO und 13 Gew.-% Na-Methacrylat
Polymerisation bei pH 2,5-3,0 (Peroxidstarter), Verfahren (A)

In einem mit Rührer, Rückflusskühler, Thermometer, Stickstoffleitung und Dosierungsleitungen ausgestatteten Glasreaktor wurde 70 g Wasser, 35 g VME-23EO, eine Teilmenge (2,0 g) von Lösung A (5,2 g Methacrylsäure gelöst in 36 g Wasser) und eine Teilmenge (0,8 g) von Lösung B (1,6 g (75% Lösung) tert-Betylperpivalat und 14,4 g iso-Propanol) vorgelegt. Die Vorlage wurde unter Stickstoff auf 75°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 75 °C gerührt. Iso-Propanol wurde abdestilliert.
Die Ergebnisse sind in Tabelle 3 zusammengefasst.

### Beispiel 4d:

Copolymer aus 89 Gew.-% VME-23EO und 11 Gew.-% Na-Methacrylat
Polymerisation bei pH 7,5-8,0 (Azo-Starter), Verfahren (B)

In einem mit Rührer, Rückflußkühler, Thermometer, Stickstoffleitung und Dosierungsleitungen ausgestatteten Glasreaktor wurde 66 g Wasser, eine Teilmenge (2,9 g) von Lösung A (35,6g VME-23EO, 4,4 g Methacrylsäure gelöst in 18g Wasser) und eine Teilmenge (1,9 g) von Lösung B (1,6 2,2'-Azobis[2-(2-imidazolin-2-yl)propane]dihydrochlorid (Wako VA-44) und 36 g Wasser) vorgelegt. Die Vorlage wurde unter Stickstoff auf 70°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 70 °C gerührt.
Die Ergebnisse sind in Tabelle 3 zusammengefasst.

### Beispiel 4e:

Copolymer aus 89 Gew.-% VME-23EO und 11 Gew.-% Na-Methacrylat
Polymerisation bei pH 7,5-8,0 (Peroxidstarter), Verfahren (B)

In einem mit Rührer, Rückflußkühler, Thermometer, Stickstoffleitung und Dosierungsleitungen ausgestatteten Glasreaktor wurde 66 g Wasser, eine Teilmenge (2,9 g) von Lösung A (35,6 g VME-23EO, 4,4 g Methacrylsäure gelöst in 18 g Wasser) und eine Teilmenge (1,9 g) von Lösung B (0,8 g (75% Lösung) tert-Betylperpivalat und 36 g iso-Propanol) vorgelegt. Die Vorlage wurde unter Stickstoff auf 75°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 75 °C gerührt. Iso-Propanol wurde abdestilliert.
Die Ergebnisse sind in Tabelle 3 zusammengefasst.

### Vergleichsbeispiel 4f:

Copolymer aus 87 Gew.-% HBVE-23EO und 13 Gew.-% Na-Methacrylat
Polymerisation bei pH 7,5-8,0 (Azo-Starter), Verfahren (A)

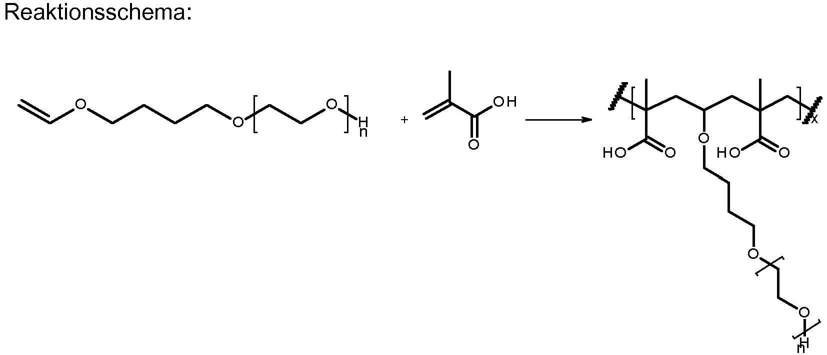

In einem mit Rührer, Rückflusskühler, Thermometer, Stickstoffleitung und Dosierungsleitungen ausgestatteten Glasreaktor wurde 57,6 g Wasser, 35 g HBVE-24EO, eine Teilmenge (1,5 g) von Lösung A (17 g 30%-ge Methacrylsäure Na-Salz Lösung und12 g Wasser) und eine Teilmenge (2,0 g) von Lösung B (1,6 g 2,2'-Azobis[2-(2-imidazolin-2-yl)propane]dihydrochlorid (Wako VA-44) und 38,4 g Wasser) vorgelegt. Die Vorlage wurde unter Stickstoff auf 70°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 70 °C gerührt.
Die Ergebnisse sind in Tabelle 3 zusammengefasst.

### Vergleichsbeispiel 4g:

Copolymer aus 87 Gew.-% HBVE-23EO und 13 Gew.-% Na-Methacrylat
Polymerisation bei pH 7,5-8,0 (Azo-Starter), Verfahren (B)

In einem mit Rührer, Rückflußkühler, Thermometer, Stickstoffleitung und Dosierungsleitungen ausgestatteten Glasreaktor wurde 66 g Wasser, eine Teilmenge (2,9 g) von Lösung A (35,6 g VME-23EO , 4,4 g Methacrylsäure gelöst in 18g Wasser) und eine Teilmenge (1,9 g) von Lösung B (1,6 2,2'-Azobis[2-(2-imidazolin-2-yl)propane]dihydrochlorid (Wako VA-44) und 36 g Wasser) vorgelegt. Die Vorlage wurde unter Stickstoff auf 70°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 70 °C gerührt.
Die Ergebnisse sind in Tabelle 3 zusammengefasst.

### Beispiel 4 h:

Copolymer aus 87 Gew.-% VME-24 EO und 13 Gew.-% Na-Methacrylat
Polymerisation bei pH 7,5-8,0 (Peroxidstarter)

In einem mit Rührer, Rückflußkühler, Thermometer, Stickstoffleitung und Dosierungsleitungen ausgestatteten Glasreaktor wurde 66 g Wasser, eine Teilmenge (2,9 g) von Lösung A (35,6 g VME-23EO, 4,4 g Acrylsäure gelöst in 18 g Wasser) und eine Teilmenge (1,9 g) von Lösung B (0,8 g (75% Lösung) tert-Betylperpivalat und 36 g iso-Propanol) vorgelegt. Die Vorlage wurde unter Stickstoff auf 75°C aufgeheizt und die Zugabe von Restmengen der Lösungen A und B gestartet, wobei die Lösung A in 3 Stunden und die Lösung B in 4 Stunden zugegeben wurde. Anschließend wurde das Reaktionsmischung 2 Stunden bei 75 °C gerührt. Iso-Propanol wurde abdestilliert.
Die Ergebnisse sind in Tabelle 3 zusammengefasst.

**Tabelle 3: Ergebnisse der Beispiele 4a - 4h**

| **Nr.** | **Makromonomer** | **Verfahren** | **Initiator** | **Temp. [°C]** | **pH** | **Mₙ [g/mol)** | **M_{w} [g/mol)** | **Mw/Mn** | **Nicht umgesetztes Makromonomer [%]*** |
|---|---|---|---|---|---|---|---|---|---|
| Beispiel 4a | VME-24EO | (A) | 4%W-VA44 | 70 | 8,3 | 24789 | 33344 | 1,34 | |
| Beispiel 4b | VME-24EO | (A) | 4%Perpivalat | 75 | 7,9 | 17980 | 41600 | 2,31 | - |
| Beispiel 4c | VME-24EO | (A) | 4%Perpivalat | 75 | 2,4 | 43381 | 68891 | 1,59 | - |
| Vergleichsbeispiel 4d | HBVE-24EO | (A) | 4%W-VA44 | 70 | 7,8 | 18408 | 24217 | 1,32 | - |
| Vergleichsbeispiel 4e | HBVE-24EO | (B) | 4%Perpivalat | 75 | 8,8 | 17271 | 23080 | 1,34 | ∼75 |
| Beispiel 4f | VME-24EO | (B) | 4%Perpivalat | 75 | 8,5 | 9054 | 20575 | 2,75 | ∼48 |
| Vergleichsbeispiel 4g | HBVE-24EO | (B) | 4%W-VA44 | 70 | 8,4 | 16875 | 24364 | 1,44 | ∼20 |
| Beispiel 4h | VME-24EO | (B) | 4%W-VA44 | 70 | 8,6 | 7968 | 14725 | 1,85 | ∼6 |

### Beispiel 5:

### Copolymerisation von VME-Alkoxylaten mit HEMA und HPMA-Phosphat im Vergleich zu HBVE Alkoxylaten

### Beispiel 5a:

### Copolymer aus 80 Gew.-% VME-67EO und 20 Gew.-% Hydroxyethylmethacrylat-phosphat

Die Versuchsapparatur besteht aus 1000 ml Doppelwandreaktor, Thermostat, Rührmotor mit Flügelrührer, Temperaturfühler, pH-Sonde und N₂-Zuleitung. In den Reaktor werden 172,80 g Wasser und 103,38 g VME-67 EO gegeben. Anschließend wird N₂ eingeleitet und der Sauerstoff verdrängt. Der Thermostat wird auf T = 75°C gestellt und der Reaktorinhalt aufgeheizt. Bei ca. 60°C erfolgt die Zugabe von 25,94 g Hydroxyethylmethacrylat-Phosphat (HEMA-P) in 137,12 g Wasser. Es stellt sich ein pH-Wert von ca. 1,0 -1,5 ein. Danach werden 9,02 g 50%ige NaOH dazugegeben um einen pH Wert von ca. 3 einzustellen. Bei der Zugabe der HEMA-P-Lösung fällt die Temperatur bis auf 50°C. Anschließend wird der Reaktorinhalt auf 60°C aufgeheizt. Daraufhin werden 1,32 g Wako VA-044 (2,2'-Azobis[2-(2-imidazolin-2-yl)propan]dihydrochlorid) in 13,2 g Wasser hinzugefügt. Nach 3 h Reaktionszeit wird der Reaktorinhalt auf 25°C abgekühlt.
Das entstandene gelbliche leicht trübe Produkt hat einen pH Wert von ca. 2,5 und einen Feststoffgehalt von 30%. Die mittlere Molmasse des Polymers(Mw) beträgt 29.000 g/mol. Die Polydispersität 1,25. Der Umsatz liegt bei ca. 75% Polymer (Bestimmung per GPC).
Die Ergebnisse sind in Tabelle 4 zusammengefasst.

### Beispiel 5b:

### Copolymer aus 84 Gew.-% VME-135EO und 16 Gew.-% Hydroxyethylmethacrylat-phosphat

Es wurde die gleiche Apparatur eingesetzt wie für Beispiel 5a.
In den Reaktor werden 172,80 g Wasser und 106,38 g VME-135 EO gegeben. Anschließend wird N₂ eingeleitet und der Sauerstoff verdrängt. Der Thermostat wird auf T = 75°C gestellt und der Reaktorinhalt aufgeheizt. Bei ca. 60°C erfolgt die Zugabe von 19,79 g Hydroxyethylmethacrylat-Phosphat (HEMA-P) in 104,6 g Wasser. Es stellt sich ein pH-Wert von ca. 1,0 -1,5 ein. Danach werden 7,05 g 50%ige NaOH dazugegeben um einen pH Wert von ca. 3 einzustellen. Bei der Zugabe der HEMA-P-Lösung fällt die Temperatur bis auf 50°C. Anschließend wird der Reaktorinhalt auf 60°C aufgeheizt. Daraufhin werden 1,26 g Wako VA-044 (2,2'-Azobis[2-(2-imidazolin-2-yl)propan]dihydrochlorid) in 11,3 g Wasser hinzugefügt.
Nach 3 h Reaktionszeit wird der Reaktorinhalt auf 25°C abgekühlt.
Die Ergebnisse sind in Tabelle 4 zusammengefasst.

### Beispiel 5c:

### Copolymer aus 89 Gew.-% VME-135EO und 11 Gew.-% Hydroxyethylmethacrylat-phosphat

Es wurde die gleiche Apparatur eingesetzt wie für Beispiel 5a.
In den Reaktor werden 328,3 g Wasser und 202,12 g VME-135 EO gegeben. Anschließend wird N₂ eingeleitet und der Sauerstoff verdrängt. Der Thermostat wird auf T = 75°C gestellt und der Reaktorinhalt aufgeheizt.Bei ca. 60°C erfolgt die Zugabe von 25,06 g Hydroxyethylmethacrylat-Phosphat (HEMA-P) in 132,5 g Wasser. Es stellt sich ein pH-Wert von ca. 1,0 -1,5 ein. Danach werden 8,90 g 50%ige NaOH dazugegeben um einen pH Wert von ca. 3 einzustellen. Bei der Zugabe der HEMA-P-Lösung fällt die Temperatur bis auf 50°C. Anschließend wird der Reaktorinhalt auf 60°C aufgeheizt. Daraufhin werden 2,27 g Wako VA-044 (2,2'-Azobis[2-(2-imidazolin-2-yl)propan]dihydrochlorid) in 21,6 g Wasser hinzugefügt.
Nach 3 h Reaktionszeit wird der Reaktorinhalt auf 25°C abgekühlt.
Die Ergebnisse sind in Tabelle 4 zusammengefasst.

### Beispiel 5d:

### Copolymer aus 92 Gew.-% VME-135EO und 8 Gew.-% Hydroxyethylmethacrylat-phosphat

Es wurde die gleiche Apparatur eingesetzt wie für Beispiel 5a.
In den Reaktor werden 328,3 g Wasser und 202,12 g VME-135 EO gegeben. Anschließend wird N₂ eingeleitet und der Sauerstoff verdrängt. Der Thermostat wird auf T = 75°C gestellt und der Reaktorinhalt aufgeheizt.Bei ca. 60°C erfolgt die Zugabe von 16,71 g Hydroxyethylmethacrylat-Phosphat (HEMA-P) in 88,31 g Wasser. Es stellt sich ein pH-Wert von ca. 1,0 -1,5 ein. Danach werden 5,78 g 50%ige NaOH dazugegeben um einen pH Wert von ca. 3 einzustellen. Bei der Zugabe der HEMA-P-Lösung fällt die Temperatur bis auf 50°C. Anschließend wird der Reaktorinhalt auf 60°C aufgeheizt. Daraufhin werden 2,19 g Wako VA-044 (2,2'-Azobis[2-(2-imidazolin-2-yl)propan]dihydrochlorid) in 21,6 g Wasser hinzugefügt.
Nach 3 h Reaktionszeit wird der Reaktorinhalt auf 25°C abgekühlt.
Die Ergebnisse sind in Tabelle 4 zusammengefasst.

### Beispiel 5e:

### Copolymer aus 84 Gew.-% VME-10PO-125EO und 16 Gew.-% Hydroxyethylmethacrylat-phosphat

Es wurde die gleiche Apparatur eingesetzt wie für Beispiel 5a.
In den Reaktor werden 328,3 g Wasser und 202,12 g VME-10 PO-125 EO gegeben. Anschließend wird N₂ eingeleitet und der Sauerstoff verdrängt. Der Thermostat wird auf T = 75°C gestellt und der Reaktorinhalt aufgeheizt.Bei ca. 60°C erfolgt die Zugabe von 37,59 g Hydroxyethylmethacrylat-Phosphat (HEMA-P) in 198,7 g Wasser. Es stellt sich ein pH-Wert von ca. 1,0 -1,5 ein. Danach werden 13,45 g 50%ige NaOH dazugegeben um einen pH Wert von ca. 3 einzustellen. Bei der Zugabe der HEMA-P-Lösung fällt die Temperatur bis auf 50°C. Anschließend wird der Reaktorinhalt auf 60°C aufgeheizt. Daraufhin werden 2,4 g Wako VA-044 (2,2'-Azobis[2-(2-imidazolin-2-yl)propan]dihydrochlorid) in 21,6 g Wasser hinzugefügt.
Nach 3 h Reaktionszeit wird der Reaktorinhalt auf 25°C abgekühlt.
Die Ergebnisse sind in Tabelle 4 zusammengefasst.

### Beispiel 5f:

### Copolymer aus 84 Gew.-% VME-135EO und 16 Gew.-% Hydroxypropylmethacrylat-phosphat

Es wurde die gleiche Apparatur eingesetzt wie für Beispiel 5a.
In den Reaktor werden 328,3 g Wasser und 202,12 g VME-135 EO gegeben. Anschließend wird N2 eingeleitet und der Sauerstoff verdrängt. Der Thermostat wird auf T = 75°C gestellt und der Reaktorinhalt aufgeheizt.Bei ca. 60°C erfolgt die Zugabe von 38,81 g Hydroxypropylmethacrylat-Phosphat (HPMA-P) in 198,7 g Wasser. Es stellt sich ein pH-Wert von ca. 1,0 -1,5 ein. Danach werden 11,5g 50%ige NaOH dazugegeben um einen pH Wert von ca. 3 einzustellen. Bei der Zugabe der HPMA-P-Lösung fällt die Temperatur bis auf 50°C. Anschließend wird der Reaktorinhalt auf 60°C aufgeheizt. Daraufhin werden 2,4 g Wako VA- 044 (2,2'-Azobis[2-(2-imidazolin-2-yl)propan]dihydrochlorid) in 21,6 g Wasser hinzugefügt.
Nach 3 h Reaktionszeit wird der Reaktorinhalt auf 25°C abgekühlt.
Die Ergebnisse sind in Tabelle 4 zusammengefasst.

### Beispiel 5g:

### Copolymer aus 98 Gew.-% VME-135EO und 2 Gew.-% Maleinsäureanhydrid

Es wurde die gleiche Apparatur eingesetzt wie für Beispiel 5a.
In den Reaktor werden 218,88 g Wasser und 134,75 g VME-135 EO gegeben. Anschließend wird N2 eingeleitet und der Sauerstoff verdrängt. Der Thermostat wird auf T = 75°C gestellt und der Reaktorinhalt aufgeheizt.Bei ca. 60°C erfolgt die Zugabe von 2,67 g Maleinsäureanhydrid (MSA) in 7,9 g Wasser. Danach werden 2,68 g 40%ige KOH dazugegeben um einen pH Wert von ca. 3 einzustellen. Bei der Zugabe der MSA-Lösung fällt die Temperatur bis auf 50°C. Anschließend wird der Reaktorinhalt auf 60°C aufgeheizt. Daraufhin werden 1,37 g Wako VA- 044 (2,2'-Azobis[2-(2-imidazolin-2-yl)propan]dihydrochlorid) in 12,37 g Wasser hinzugefügt.
Nach 3 h Reaktionszeit wird der Reaktorinhalt auf 25°C abgekühlt.
Die Ergebnisse sind in Tabelle 4 zusammengefasst.

### Vergleichsbeispiel 5a:

### Copolymer aus 84 Gew.-% HBVE-135EO und 16 Gew.-% Hydroxyethylmethacrylat-phosphat

Es wurde die gleiche Apparatur eingesetzt wie für Beispiel 5a.
In den Reaktor werden 217,15 g Wasser und 133,68 g HBVE-135EO gegeben. Anschließend wird N₂ eingeleitet und der Sauerstoff verdrängt. Der Thermostat wird auf T = 75°C gestellt und der Reaktorinhalt aufgeheizt.Bei ca. 60°C erfolgt die Zugabe von 25,72 g Hydroxyethylmethacrylat-Phosphat (HEMA-P) in 135,95 g Wasser. Danach werden 9,13 g 50%ige NaOH dazugegeben um einen pH Wert von ca. 6,5 einzustellen. Bei der Zugabe der HEMA-P-Lösung fällt die Temperatur bis auf 50°C. Anschließend wird der Reaktorinhalt auf 60°C aufgeheizt. Daraufhin werden 1,59 g Wako VA-044 (2,2'-Azobis[2-(2-imidazolin-2-yl)propan]dihydrochlorid) in 11,34 g Wasser hinzugefügt.
Nach 3 h Reaktionszeit wird der Reaktorinhalt auf 25°C abgekühlt.
Die Ergebnisse sind in Tabelle 4 zusammengefasst.

### Vergleichsbeispiel 5b:

### Copolymer aus 89 Gew.-% HBVE-135EO und 11 Gew.-% Hydroxyethylmethacrylat-phosphat

Es wurde die gleiche Apparatur eingesetzt wie für Beispiel 5a.
In den Reaktor werden 217,15 g Wasser und 133,68 g HBVE-135EO gegeben. Anschließend wird N2 eingeleitet und der Sauerstoff verdrängt. Der Thermostat wird auf T = 75°C gestellt und der Reaktorinhalt aufgeheizt. Bei ca. 60°C erfolgt die Zugabe von 17,15 g Hydroxyethylmethacrylat-Phosphat (HEMA-P) in 90,64 g Wasser. Danach werden 9,5 g 50%ige NaOH dazugegeben um einen pH Wert von ca. 6.5 einzustellen. Bei der Zugabe der HEMA-P-Lösung fällt die Temperatur bis auf 50°C. Anschließend wird der Reaktorinhalt auf 60°C aufgeheizt. Daraufhin werden 1,51 g Wako VA-044 (2,2'-Azobis[2-(2-imidazolin-2-yl)propan]dihydrochlorid) in 11,34 g Wasser hinzugefügt. Nach 3 h Reaktionszeit wird der Reaktorinhalt auf 25°C abgekühlt.
Die Ergebnisse sind in Tabelle 4 zusammengefasst.

### Vergleichsbeispiel 5c:

### Copolymer aus 98 Gew.-% HBVE-135EO und 2 Gew.-% Maleinsäureanhydrid

Es wurde die gleiche Apparatur eingesetzt wie für Beispiel 5a.
In den Reaktor werden 434,3 g Wasser und 267,37 g HBVE-135EO gegeben. Anschließend wird N2 eingeleitet und der Sauerstoff verdrängt. Der Thermostat wird auf T = 75°C gestellt und der Reaktorinhalt aufgeheizt. Bei ca. 60°C erfolgt die Zugabe von 5,48 g Maleinsäureanhydrid (MSA) in 28,62 g Wasser. Danach werden 5,3 g 40%ige KOH dazugegeben um einen pH Wert von ca. 6.5 einzustellen. Bei der Zugabe der MSA-Lösung fällt die Temperatur bis auf 50°C. Anschließend wird der Reaktorinhalt auf 60°C aufgeheizt. Daraufhin werden 2,73 g Wako VA-044 (2,2'-Azobis[2-(2-imidazolin-2-yl)propan]dihydrochlorid) in 24,55 g Wasser hinzugefügt. Nach 3 h Reaktionszeit wird der Reaktorinhalt auf 25°C abgekühlt.
Die Ergebnisse sind in Tabelle 4 zusammengefasst.

**Tabelle 4: Ergebnisse der Beispiele 5a bis 5g sowie der Vergleichsbeispiele 5a bis 5c (* Umsatz bestimmt via GPC)**

| **Beispiel** | **Makromonomer** | **Gewichtsverhältnis Makromonomer / Comonomer** | **Mₙ [g/mol]** | **M_{w} [g/mol]** | **PD** | **Feststoff [Gew.-%]** | **Umsatz* [%]** |
|---|---|---|---|---|---|---|---|
| Beispiel 5a | VME-67EO | 80 / 20 (HEMA-P) | 22100 | 27400 | 1,24 | 30 | 93 |
| Beispiel 5b | VME-135EO | 84 / 16 (HEMA-P) | 19000 | 25600 | 1,35 | 33 | 78 |
| Beispiel 5c | VME 135EO | 89 / 11 (HEMA-P) | 19900 | 27600 | 1,39 | 32 | 82 |
| Beispiel 5d | VME-135EO | 92 / 8 (HEMA-P) | 50593 | 91007 | 1,79 | 33 | 70 |
| Beispiel 5e | VME-10PO-125EO | 84 / 16 (HEMA-P) | 55000 | 80300 | 1,46 | 31 | 81 |
| Beispiel 5f | VME-135EO | 84 / 16 (HEMA-P) | 17100 | 21900 | 1,28 | 31 | 74 |
| Beispiel 5g | VME-135EO | 98 / 2 (MSA) | 19700 | 25400 | 1,29 | 37 | 62 |
| Vergleichsbeispiel 5 a | HBVE-135EO | 84 / 16 (HEMA-P) | 32200 | 84600 | 2,63 | 31 | 6 |
| Vergleichsbeispiel 5 b | HBVE-135EO | 89 / 11 (HEMA-P) | 23900 | 34500 | 1,44 | 33 | 5 |
| Vergleichsbeispiel 5 c | HBVE-135EO | 98 / 2 (MSA) | - | - | - | 37 | 0 |

### Anwendungstests

### Testmethode:

### Ausbreittests mit Polycarboxylatetherverflüssigern auf Basis VME-Ethoxylat im Vergleich zu HBVE-Ethoxylat

Es wurde ein Mörtel aus 450 g Heidelberger Zement CEM I, 42,5 R, 1350 g Normsand und 225 g Wasser (abzüglich des mit dem Verflüssiger zuzugebendem Wasser) hergestellt, gemäß DIN EN 196-1. Der Verflüssiger (zwischen 0,1 und 0,3 Gew.% bzg. auf dem Zement) wurde zusammen mit demEntschäumer Tri-isobutylphosphat (7 Gew.% bezogen auf die Trockenmasse des Verflüssigers) nach 90 Sekunden während der Mörtelzubereitung zugegeben. Nach der Zugabe wurde noch 60 s gemischt. Anschließend wurde der Mörtel in eine konische Metallform gefüllt, die mittig auf einem Schocktisch an dem Ursprung eines rechtwinkligen Koordinatensystems mit zwei Achsen (cm-Einheiten) platziert wurde. Überschüssiger Mörtel am oberen Rand wurde abgestreift und die Form abgehoben, so dass ein konischer Mörtelkuchen auf dem Ausbreittisch zurückblieb. Nach 15 Schlägen (Hubstöße) durch den Schocktisch wurde der Durchmesser des Kuchens entlang der Achsen bestimmt. Je größer der Durchmesser des Mörtelkuchens, umso besser die Fließeigenschaften des Mörtel. Die Messung wurde mit derselben Mörtelprobe nach 30, 60 und 90 Minuten wiederholt. Die Temperatur betrug 23 +/-1 °C.

Die jeweils verwendeten Polymere, deren Mengen sowie die Testergebnisse sind in Tabelle 5 zusammengefasst.

**Tabelle 5: Ergebnisse der anwendungstechnischen Tests**

| **Nr.** | **Verfahren** | **Polymer Nr.** | **Art des Polymers** | **pH-Wert bei Polymerisation** | **Mₙ [g/mol]** | **M_{w} [g/mol]** | **M_{w}/Mₙ** | **Menge Polymer bzgl. Zement [Gew.-%]** | **Durchmesser nach x Min. [cm]** | | | |
|---|---|---|---|---|---|---|---|---|---|---|---|---|
| | | | | | | | | | **1 Min.** | **30 Min.** | **60 Min.** | **90 Min.** |
| V1 | (A) | - | Nullprobe ohne Polymer | - | - | - | - | - | 17,65 | 15,7 | 14,95 | 13,35 |
| V2 | (A) | V2a | 89 % HBVE-24EO, 11% AS | 2-3 | 1267 | 12459 | 9,83 | 0,12 | 19,55 | 17,2 | 15,4 | 15,4 |
| V3 | (A) | V2b | 89 % HBVE-24EO, 11% AS | 2-3 | 1141 | 4366 | 3,82 | 0,12 | 19,3 | 17,5 | 16,8 | 14,85 |
| V4 | (A) | V2c | 89 %HBVE-24EO, 11 % AS | 2-3 | 1478 | 17994 | 12,2 | 0,12 | 19,55 | 17,6 | 16,75 | 15,7 |
| 1 | (A) | 2a | 89 % VME-23EO , 11 % AS | 2-3 | 6348 | 34566 | 5,44 | 0,12 | 20,7 | 18,55 | 16,75 | 15,9 |
| 2 | (A) | 2b | 89 % VME-23EO , 11 % AS | 2-3 | 6137 | 41648 | 6,78 | 0,12 | 21,15 | 18,25 | 17,2 | 16,45 |
| 3 | (A) | 2c | 89 % VME-23EO , 11 % AS | 2-3 | 6591 | 42874 | 6,5 | 0,12 | 21,05 | 18,35 | 16,85 | 16,25 |
| V5 | (A) | V2c | 89 %HBVE-24EO, 11 % AS | 2-3 | 1478 | 17994 | 12,2 | 0,24 | 21,1 | 19,45 | 18,4 | 16,3 |
| 4 | (A) | 2b | 89 % VME-23EO , 11 % AS | 2-3 | 6137 | 41648 | 6,78 | 0,24 | 26 | 22,2 | 19,6 | 18,35 |

**Tabelle 5 (Fortsetzung): Ergebnisse der anwendungstechnischen Tests**

| **Nr.** | **Verfahren** | **Polymer Nr.** | **Art des Polymers** | **pH-Wert bei Polymerisation** | **Mₙ [g/mol]** | **M_{w} [g/mol]** | **M_{w}/Mₙ** | **Menge Polymer bzgl. Zement [Gew.-%]** | **Durchmesser nach x Min. [cm]** | | | |
|---|---|---|---|---|---|---|---|---|---|---|---|---|
| | | | | | | | | | **1 Min.** | **30 Min.** | **60 Min.** | **90 Min.** |
| 5 | (A) | 2d | 85 % VME-23 EO, 11 % AS | 2-3 | 4974 | 20834 | 4,18 | 0,115 | 20,55 | 18,35 | 17,55 | 16,65 |
| 6 | (A) | 2d | 85 % VME-23 EO, 11 % AS | 7-8 | 4974 | 20834 | 4,18 | 0,23 | 23,15 | 19,45 | 18,4 | 17,5 |
| 7 | (B) | 3a | 89 % VME-23EO, 11 % AS | 2-3 | 27709 | 42697 | 2,7 | 0,1 | 29,3 | 24,9 | 22,25 | 21,3 |
| 8 | (B) | V3a | 86 % HBVE-24EO, 14 % AS | 8-9 | 12238 | 15404 | 1,26 | 0,12 | 21,1 | 18,7 | 17,5 | 16,25 |
| V6 | (B) | V3b | 86 % HBVE-24EO, 14 % AS | 7-9 | 18028 | 25349 | 1,41 | 0,12 | 22,5 | 19,1 | 17,3 | 16,6 |
| 9 | (B) | 3b | 89 % VME-23EO, 11 % AS | 2-3 | 23197 | 37150 | 2,7 | 0,1 | 28,05 | 23,15 | 22,25 | 19,55 |
| 10 | (B) | 3c | 97 % VME-135EO, 3 % AS | 8- 10 | 15682 | 20930 | 1,33 | 0,11 | 19,2 | 16,8 | 15,65 | 15,15 |
| 11 | (B) | V3c | 94 % HBVE-135EO, 6 % AS | 7-9 | 36819 | 39428 | 1,07 | 0,11 | 19,3 | 16,9 | 16 | 15 |
| 12 | (B) | 3d | 97 % VME-135EO, 3 % AS | 8-10 | 18776 | 29705 | 1,58 | 0,11 | 19 | 16,95 | 15,95 | 15,1 |

**Tabelle 5 (Fortsetzung): Ergebnisse der anwendungstechnischen Tests**

| **Nr.** | **Verfahren** | **Polymer Nr.** | **Art des Polymers** | **pH-Wert bei Polymerisation** | **Mₙ [g/mol]** | **M_{w} [g/mol]** | **M_{w}/Mₙ** | **Menge Polymer bzgl. Zement [Gew.-%]** | **Durchmesser nach x Min. [cm]** | | | |
|---|---|---|---|---|---|---|---|---|---|---|---|---|
| | | | | | | | | | **1 Min.** | **30 Min.** | **60 Min.** | **90 Min.** |
| 13 | (A) | 4a | VME-24EO, MAS | 8-9 | 24789 | 33344 | 1,34 | 0,11 | 21,7 | 18,45 | 17,95 | 17,65 |
| 14 | (A) | 4b | VME-24EO, MAS | 8-9 | 17980 | 41600 | 2,31 | 0,11 | 27,3 | 21,85 | 18,60 | 16,45 |
| 15 | (A) | 4c | VME-24EO, MAS | 2-3 | 43381 | 68891 | 1,59 | 0,11 | 21,35 | 18,35 | 17,55 | 16,25 |
| V7 | (A) | V4d | HBVE-24EO, MAS | 7,8 | 18408 | 24217 | 1,32 | 0,11 | 19,7 | 18 | 17 | 16,25 |
| V8 | (B) | | HBVE-24EO, MAS | 8-9 | 14585 | 19709 | 1,35 | 0,11 | 19,5 | 17,2 | 16,65 | 16,35 |
| 16 | (B) | | VME-24EO, MAS | 8-9 | 27877 | 40371 | 1,45 | 0,11 | 21,85 | 18,75 | 17,6 | 16,85 |
| V9 | (B) | V4e | HBVE-24EO, MAS | 8-9 | 17271 | 23080 | 1,34 | 0,11 | 20,35 | 17,8 | 16,55 | 15,45 |
| 16 | (B) | 4f | VME-24EO, MAS | 8-9 | 9054 | 20575 | 2,75 | 0,11 | 20,95 | 17,70 | 16,75 | 15,80 |
| V10 | (B) | V4g | HBVE-24EO, MAS | 8-9 | 16875 | 24364 | 1,44 | 0,11 | 20,45 | 17,60 | 16,70 | 16,00 |
| 17 | (B) | 4h | VME-24EO, MAS | 8-9 | 7968 | 14725 | 1,85 | 0,11 | 20,15 | 17,50 | 16,30 | 15,85 |

### Kommentar zu den anwendungstechnischen Versuchen

Die Beispiele in Tabelle zeigen, dass die Versuche mit den VME-Ethoxylaten eine bessere Wirkung im Hinblick auf die Mörtelverflüssigung aufweisen, als die HBVE-basierten Versuche.

Im Nullversuch betrug der Durchmesser des Mörtelkuchens nach 1 min 17,65 cm. Der Zusatz von 0,12 % eines HBVE-basierten Verflüssigers (Vergleichsbeispiel V2) führt nach 1 min zu einem Mörtelkuchen von 19,55 cm Durchmesser. Mit dem entsprechenden VME-basierten Produkt (Beispiel 2) werden 21,15 cm erreicht. Auch bei einer Verdoppelung der Konzentration ist das VME-basierte Produkt besser; es werden 26 cm (Versuch 4) mit dem VME-basierten Produkt erzielt, mit dem HBVE-basierten Produkt nur 21,1 cm.

Die Versuche zeigen außerdem, dass mit gemäß Verfahren (B) hergestellten Polymeren bessere anwendungstechnische Ergebnisse erzielt werden, als mit gemäß Verfahren (A) hergestellten Polymeren: Bei einer Menge von 0,12 Gew.-% ergab der beste Versuch mit einem gemäß Verfahren (A) hergestellten VME-basierten Produkt 21,15 cm (Versuch 2). Bei den Versuchen 7 und 9 mit einem gemäß Verfahren (B) hergestellten VME-basierten-Produkt wurden ca. 28 bis 29 cm erzielt, obwohl die Polymermenge mit 0,1 Gew.-% sogar geringer war als bei Versuch 2 (0,12 Gew.-%).

Ohne dass wir damit auf eine Theorie festgelegt werden möchten, scheint dies darauf zurückzuführen zu sein, dass die Verbindungen (I) aufgrund ihrer relativ hohen Reaktivität relativ leicht mit sich selbst polymerisieren. Da bei Verfahren (A) die Verbindungen (I) vorgelegt und die Comonomere nach und nach zugegeben werden, wird die Bildung von Blockstrukturen begünstigt. Verfahren (B), bei dem beide Monomere nach und nach zugegeben werden, scheint zu einem gleichmäßigeren Einbau der VME-Monomere in das Copolymer zu führen. Dadurch lässt sich offensichtlich eine deutlich bessere Wirkung als Betonverflüssiger erzielen zu lassen.

## Patentansprüche

1. Ungesättigte Verbindungen der allgemeinen Formel (I) 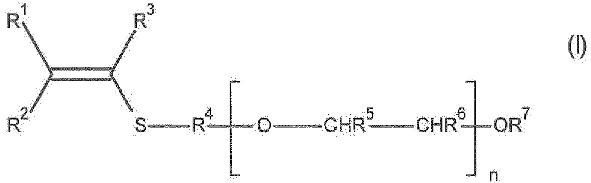 wobei
R¹, R², R³ unabhängig voneinander, gleich oder verschieden, H, CH₃,
R⁴ lineares oder verzweigtes C₁-C₃₀-Alkylen,
R⁵, R⁶ unabhängig voneinander, gleich oder verschieden, H, C₁-C₂₀-Alkyl, C₃-C₁₅-Cycloalkyl, Aryl, -CH₂-O-C₁-C₂₀-Alkyl, CH₂-O-C₂-C₂₀-Alkenyl, wobei R⁵ und R⁶ auch gemeinsam ein C₃-C₆-Alkylen bilden können,
R⁷ unabhängig voneinander, gleich oder verschieden, H, C₁-C₄-Alkyl, 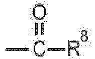
R⁸ C₁-C₂₂-Alkyl, C₂-C₂₂-Alkenyl, und
n eine ganze Zahl von 2 bis 200
bedeuten.

2. Ungesättigte Verbindungen gemäß Anspruch 1, **dadurch gekennzeichnet, dass** R⁴ für eine C₂-C₄-Alkylengruppe, R⁵, R⁶ unabhängig voneinander, gleich oder verschieden für eine Gruppe ausgewählt aus H, -CH₃, -CH₂-CH₃, -C₃-C₁₁-Alkyl, C₁₂-C₂₂-Alkyl, Phenyl, -CH₂-O-C₁-C₁₀-Alkyl, CH₂-O-C₂-C₁₀-Alkenyl, R⁷ für H oder C₁-C₄-Alkyl und n für 5 bis 140 stehen.

3. Ungesättigte Verbindungen gemäß Anspruch 1, **dadurch gekennzeichnet, dass** es sich um ungesättigte Verbindungen (Ia) 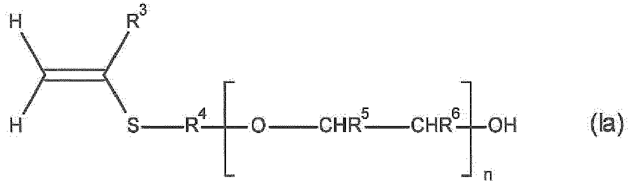 handelt, wobei
R³ für H oder Methyl,
R⁴ für eine lineare oder verzweigte C₂-C₁₀-Alkylengruppe,
R⁵, R⁶ unabhängig voneinander für H, Methyl oder Ethyl stehen, mit der Maßgabe, dass die Summe der Kohlenstoffatome in den Resten R⁵ und R⁶ pro Alkoxygruppe jeweils 0 bis 2 beträgt, und
n für 5 bis 160 steht.

4. Ungesättigte Verbindungen gemäß Anspruch 1, **dadurch gekennzeichnet, dass** es sich um ungesättigte Verbindungen (Ib) 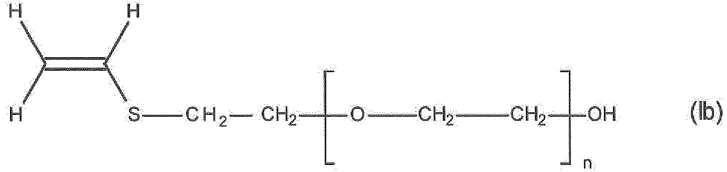 handelt, wobei n für 20 bis 140 steht.

5. Ungesättigte Verbindungen gemäß Anspruch 4, **dadurch gekennzeichnet, dass** n für 20 bis 30 steht.

6. Mischungen, enthaltend Verbindungen der allgemeinen Formel (I) gemäß Anspruch 1.

7. Polymere, enthaltend Verbindungen der allgemeinen Formel (I) gemäß Anspruch 1 als Monomere.

8. Polymere gemäß Anspruch 7, enthaltend Verbindungen der allgemeinen Formel (Ia) gemäß Anspruch 3 als Monomere.

9. Polymere gemäß Anspruch 7, enthaltend Verbindungen der allgemeinen Formel (Ib) gemäß Anspruch 4 als Monomere.

10. Polymer gemäß einem der Ansprüche 7 bis 9, **dadurch gekennzeichnet, dass** mindestens ein weiteres von den Verbindungen der allgemeinen Formel (I), (Ib) bzw. (Ic) verschiedenes Monomer im Polymer enthalten ist.

11. Polymer gemäß Anspruch 10, **dadurch gekennzeichnet, dass** es sich bei den weiteren Monomeren um mindestens ein monoethylenisch ungesättigtes Monomer handelt.

12. Polymer gemäß Anspruch 11, **dadurch gekennzeichnet, dass** es sich bei den monoethylenisch ungesättigten Monomeren um Säuregruppen umfassende Monomere handelt, wobei die Säuregruppen auch ganz oder teilweise neutralisiert sein können.

13. Polymer gemäß Anspruch 12, **dadurch gekennzeichnet, dass** es sich bei den Säuregruppen um Gruppen ausgewählt aus der Gruppen von Carbonsäure-, Sulfonsäure-, Phophor- oder Phosphonsäuregruppen handelt.

14. Polymer gemäß Anspruch 13, **dadurch gekennzeichnet, dass** das Polymer mindestens ein Monomer mit Carbonsäuregruppen ausgewählt aus der Gruppe von Acrylsäure, Methacrylsäure, (Meth)Acrylsäureanhydrid, Crotonsäure, Maleinsäure, Maleinsäureanhydrid, Fumarsäure, Itaconsäure, Mesaconsäure, Citraconsäure oder Methylenmalonsäure bzw. Salze davon umfasst.

15. Polymer gemäß Anspruch 13, **dadurch gekennzeichnet, dass** das Polymer mindestens ein Monomer mit Phosphor- oder Phosphonsäuregruppen ausgewählt aus der Gruppe von Vinylphosphonsäure, Ester von Hydroxyethyl, Hydroxypropyl- oder Hydroxybutyl(meth)acrylat mit (Poly)Phosphorsäure, Phosphorsäuremonovinylester, Allylphosphonsäure, Phosphorsäuremonoallylester, 3-Butenylphosphonsäure, Phosphorsäure(mono-3-butenyl)ester, Phosphorsäuremono-(4-vinyloxybutyl)ester, Phosphorsäuremono-(-2-hydroxy-3-vinyloxy-propyl)ester, Phosphorsäuremono-(1-phosphonoxymethyl-2-vinyloxy-ethyl)-ester, Phosphorsäuremono-(3-allyloxy-2-hydroxy-propyl)ester, Phosphorsäuremono-2-(allylox-1-phosphonoxymethyl-ethyl)ester, 2-Hydroxy-4-vinyloxymethyl-1,3,2-dioxaphosphol, 2-Hydroxy-4-allyloxymethyl-1,3,2-dioxaphosphol bzw. Salze davon umfasst.

16. Polymer gemäß Anspruch 13, **dadurch gekennzeichnet, dass** das Polymer mindestens ein Monomer mit Sulfonsäuregruppen ausgewählt aus der Gruppe von Vinylsulfonsäure, 2-Acrylamido-2-methylpropanesulfonsäure, 2-Acrylamidomethyldodecylsulfonsäure, 2-(Meth)acryloxyethansulfonsäure, 3-(Meth)acryloxypropansulfonsäure, Allyloxybenzolsulfonsäure, Vinylbenzolsulfonsäure, Vinyltoluolsulfonsäure, Allylsulfonsäure, Methallylsulfonsäure bzw. Salze davon umfasst.

17. Polymer gemäß einem der Ansprüche 7 bis 9, dadruch gekennzeichnet, dass als mindestens ein monoethylenisch ungesättigtes Monomer Styrol, Butadien, Methyl-methacrylat, (Meth)acrylat, Ethylacrylat, Dibutylmaleat, Metyhl-alpha-cyanacrylat, Acrylnitril, Acrylsäure, Methacrylsäure, Maleinsäure(anhydrid), Itaconsäure, Vinylphosphonsäure, N-Vinylpyrrolidon, N,N-Dimetyl-N,N-diallylammoniumchlorid, Acrylamid, Vinylimidazol, Vinylalkohol, Vinylacetat, Allylsulfonsäure, 2-Acrylamido-2-methylpropan-sulfonsäure, (Iso)prenylalkoxylat, (Meth)allylalkoxylat oder Hydroxybutylvinyletheralkoxylat enthalten ist.

18. Polymer gemäß Anspruch 17, wobei als mindestens ein monoethylenisch ungesättigtes Monomer Acrylsäure, Methacrylsäure, (Meth)acrylat, Maleinsäure(anhydrid), (Iso)prenylalkoxylat, (Meth)allylalkoxylat oder Hydroxybutylvinyletheralkoxylat enthalten ist.

19. Polymer gemäß Anspruch 18, wobei neben ungesättigten Verbindungen der allgemeinen Formel (I), Acrylsäure, Methacrylsäure, (Meth)acrylat, Maleinsäure(anhydrid), (Iso)prenylalkoxylat, (Meth)allylalkoxylat oder Hydroxybutylvinyletheralkoxylat keine weiteren Monomere enthalten sind.

20. Polymer gemäß Anspruch 17, wobei neben ungesättigten Verbindungen der allgemeinen Formel (I) und Acrylsäure und Methacrylsäure oder Acrylsäure und Maleinsäure(anhydrid) oder Methacrylsäure und Maleinsäure(anhydrid) keine weiteren Monomere enthalten sind.

21. Polymer gemäß Anspruch 20, wobei neben ungesättigten Verbindungen der allgemeinen Formel (I), Acrylsäure, Methacrylsäure und Maleinsäure(anhydrid) keine weiteren Monomere vorhanden sind.

22. Polymer gemäß einem der Ansprüche 7 bis 21, **dadurch gekennzeichnet, dass** 5 bis 99,9 Gew.-% ungesättigte Verbindungen der allgemeinen Formel (I), (la) oder (Ib) bezogen auf die Gesamtmenge an Monomeren im Polymer enthalten sind.

23. Polymer gemäß einem der Ansprüche 7 bis 21, dadruch gekennzeichnet, dass 5 bis 99,9 Gew.-% ungesättigte Verbindungen der allgemeinen Formel (I) und von 95 bis 0,1 Gew.-% weitere, monoethylenisch ungesättigte Monomere jeweils bezogen auf die Gesamtmenge an Monomeren enthalten sind.

24. Polymer gemäß Anspruch 18, wobei von 5 bis 99,9 Gew.-% ungesättigte Verbindungen der allgemeinen Formel (I) und insgesamt von 95 bis 0,1 Gew.-% Acrylsäure, Methacrylsäure, (Meth)acrylat, Maleinsäure(anhydrid), (Iso)prenylalkoxylat, (Meth)allylalkoxylat oder Hydroxybutylvinyletheralkoxylat jeweils bezogen auf die Gesamtmenge an Monomeren enthalten sind.

25. Polymer gemäß einem der Ansprüche 7 bis 24, **dadurch gekennzeichnet, dass** das zahlenmittlere Molekulargewicht Mₙ der Polymere 1000 g/mol bis 1000000 g/mol beträgt.

26. Polymer gemäß einem der Ansprüche 7 bis 24, **dadurch gekennzeichnet, dass** das zahlenmittlere Molekulargewicht Mₙ der Polymere 5000 g/mol bis 100000 g/mol beträgt.

27. Verfahren zur Herstellung von Polymeren gemäß einem der Ansprüche 7 bis 26, **dadurch gekennzeichnet, dass** man ungesättigte Verbindungen (I) sowie optional mindestens ein weiteres Monomer radikalisch polymerisiert.

28. Verfahren gemäß Anspruch 27, **dadurch gekennzeichnet, dass** es sich um eine Copolymerisation handelt, bei der man ungesättigte Verbindungen (I) sowie mindestens ein monoethylenisch ungesättigtes, weiteres Monomer radikalisch polymerisiert.

29. Verfahren gemäß Anspruch 28, **dadurch gekennzeichnet, dass** es sich bei den monoethylenisch ungesättigten Monomeren um Säuregruppen umfassende Monomere handelt, wobei die Säuregruppen auch ganz oder teilweise neutralisiert sein können.

30. Verfahren gemäß einem der Ansprüche 27 bis 29, **dadurch gekennzeichnet, dass** es sich um ungesättigte Verbindungen (Ia) handelt.

31. Verfahren gemäß einem der Ansprüche 27 bis 29, **dadurch gekennzeichnet, dass** es sich um ungesättigte Verbindungen (Ib) handelt.

32. Verfahren gemäß einem der Ansprüche 27 bis 31, **dadurch gekennzeichnet, dass** man die radikalische Polymerisation in wässriger Lösung vornimmt.

33. Verfahren gemäß Anspruch 32, **dadurch gekennzeichnet, dass** der pH-Wert im Zuge der Polymerisation 1 bis 6 beträgt.

34. Verfahren gemäß Anspruch 32, **dadurch gekennzeichnet, dass** der pH-Wert im Zuge der Polymerisation 1 bis 3 beträgt.

35. Verfahren gemäß einem der Ansprüche 28 bis 34, **dadurch gekennzeichnet, dass** man die radikalische Polymerisation vornimmt, indem man einen Teil der ungesättigten Verbindungen (I), (Ia) oder (Ib), einen Teil der weiteren Monomere sowie einen Teil eines Polymerisationsinitiators in einem Reaktor vorlegt und die verbliebenen Mengen der ungesättigten Verbindungen (I), (la) oder (Ib), der weiteren Monomere sowie des Polymerisationsinitiators nach und nach in den Polymerisationsreaktor eindosiert.

36. Verfahren gemäß Anspruch 35, **dadurch gekennzeichnet, dass** die Menge der vorgelegten Monomere 25 Gew.-% der Gesamtmenge der Monomere nicht überschreitet.

37. Verwendung von Polymeren gemäß den Ansprüchen 7 bis 26 als Zementadditive, Mahlhilfe bei der Herstellung von Zement, Betonverflüssiger, Zusatz zu hydraulischen Bindemitteln, reaktive Weichmacher für die Herstellung von Kunststoffen, Kautschuk oder Latex, Assoziativverdicker, Antioxidantien, oder für die Herstellung von Polyethersiloxanen.

38. Mischungen enthalten Polymere gemäß den Ansprüchen 7 bis 26.

## Claims

1. An unsaturated compound of the general formula (I) 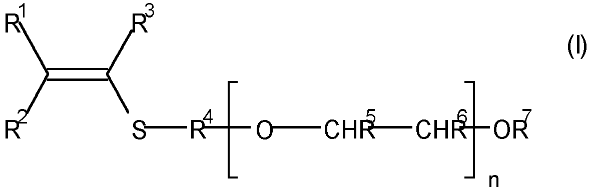 where
R¹, R², R³ are the same or different and are each independently H, CH₃,
R⁴ is a linear or branched C₁-C₃₀-alkylene,
R⁵, R⁶ are the same or different and are each independently, H, C₁-C₂₀-alkyl, C₃-C₁₅-cycloalkyl, aryl, -CH₂-O-C₁-C₂₀-alkyl, CH₂-O-C₂-C₂₀-alkenyl,
where R⁵ and R⁶ may together form a C₃-C₆-alkylene,
R⁷, is the same or different and is independently, H, C₁-C₄-alkyl, 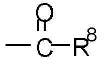
R⁸ is C₁-C₂₂-alkyl, C₂-C₂₂-alkenyl, and
n is an integer from 2 to 200.

2. The unsaturated compound according to claim 1, wherein R⁴ is a C₂-C₄-alkylene group, R⁵, R⁶ are the same or different and are each independently a group selected from H, -CH₃, -CH₂-CH₃, -C₃-C₁₁-alkyl, C₁₂-C₂₂-alkyl, phenyl, -CH₂-O-C₁-C₁₀-alkyl, CH₂-O-C₂-C₁₀-alkenyl, R⁷ is H or C₁-C₄-alkyl and n is 5 to 140.

3. The unsaturated compound according to claim 1, wherein said compound is an unsaturated compound (Ia) 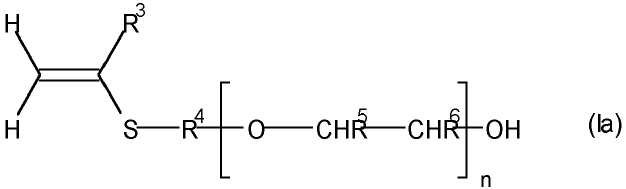 where
R³ is H or methyl,
R⁴ is a linear or branched C₂-C₁₀-alkylene group,
R⁵, R⁶ are each independently H, methyl or ethyl, with the proviso that the sum total of carbon atoms in the R⁵ and R⁶ residues per alkoxy group is in each case 0 to 2, and
n is 5 to 160.

4. The unsaturated compound according to claim 1, wherein said compound is an unsaturated compound (Ib), 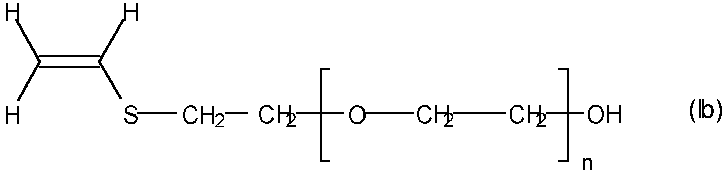 where n is 20 to 140.

5. The unsaturated compound according to claim 4, wherein n is 20 to 30.

6. A mixture comprising compounds of the general formula (I) according to claim 1.

7. A polymer comprising compounds of the general formula (I) according to claim 1 as monomers.

8. The polymer according to claim 7, comprising compounds of the general formula (Ia) according to claim 3 as monomers.

9. The polymer according to claim 7, comprising compounds of the general formula (Ib) according to claim 4 as monomers.

10. The polymer according to any of claims 7 to 9, wherein at least one further monomer different from the compounds of the general formula (I), (Ib) or (Ic) is present in the polymer.

11. The polymer according to claim 10, wherein the further monomers are at least one monoethylenically unsaturated monomer.

12. The polymer according to claim 11, wherein the monoethylenically unsaturated monomers are monomers comprising acid groups, where the acid groups may also be completely or partly neutralized.

13. The polymer according to claim 12, wherein the acid groups are groups selected from the groups of carboxylic acid groups, sulfonic acid groups, phosphoric acid groups or phosphonic acid groups.

14. The polymer according to claim 13, wherein the polymer comprises at least one monomer having carboxylic acid groups selected from the group of acrylic acid, methacrylic acid, (meth)acrylic anhydride, crotonic acid, maleic acid, maleic anhydride, fumaric acid, itaconic acid, mesaconic acid, citraconic acid or methylene malonic acid or salts thereof.

15. The polymer according to claim 13, wherein the polymer comprises at least one monomer having phosphoric acid groups or phosphonic acid groups selected from the group of vinylphosphonic acid, esters of hydroxyethyl, hydroxypropyl or hydroxybutyl (meth)acrylate with (poly)phosphoric acid, monovinyl phosphate, allylphosphonic acid, monoallyl phosphate, 3-butenylphosphonic acid, mono-3-butenyl phosphate, mono(4-vinyloxybutyl) phosphate, mono(2-hydroxy-3-vinyloxypropyl) phosphate, mono(1-phosphonoxymethyl-2-vinyloxyethyl) phosphate, mono(3-allyloxy-2-hydroxypropyl) phosphate, mono[2-(allyloxy-1-phosphonoxymethylethyl)] phosphate, 2-hydroxy-4-vinyloxymethyl-1,3,2-dioxaphosphole, 2-hydroxy-4-allyloxymethyl-1,3,2-dioxaphosphole or salts thereof.

16. The polymer according to claim 13, wherein the polymer comprises at least one monomer having sulfonic acid groups selected from the group of vinylsulfonic acid, 2-acrylamido-2-methylpropanesulfonic acid, 2-acrylamidomethyldodecylsulfonic acid, 2-(meth)acryloxyethanesulfonic acid, 3-(meth)acryloxypropanesulfonic acid, allyloxybenzenesulfonic acid, vinylbenzenesulfonic acid, vinyltoluenesulfonic acid, allylsulfonic acid, methallylsulfonic acid or salts thereof.

17. The polymer according to any of claims 7 to 9, wherein at least one monoethylenically unsaturated monomer present is styrene, butadiene, methyl methacrylate, (meth)acrylate, ethyl acrylate, dibutyl maleate, methyl alpha-cyanoacrylate, acrylonitrile, acrylic acid, methacrylic acid, maleic acid (anhydride), itaconic acid, vinylphosphonic acid, N-vinylpyrrolidone, N,N-dimethyl-N,N-diallylammonium chloride, acrylamide, vinylimidazole, vinyl alcohol, vinyl acetate, allylsulfonic acid, 2-acrylamido-2-methylpropanesulfonic acid, (iso)prenyl alkoxylate, (meth)allyl alkoxylate or hydroxybutyl vinyl ether alkoxylate.

18. The polymer according to claim 17, wherein at least one monoethylenically unsaturated monomer present is acrylic acid, methacrylic acid, (meth)acrylate, maleic acid (anhydride), (iso)prenyl alkoxylate, (meth)allyl alkoxylate or hydroxybutyl vinyl ether alkoxylate.

19. The polymer according to claim 18, wherein no other monomers are present besides unsaturated compounds of the general formula (I), acrylic acid, methacrylic acid, (meth)acrylate, maleic acid (anhydride), (iso)prenyl alkoxylate, (meth)allyl alkoxylate or hydroxybutyl vinyl ether alkoxylate.

20. The polymer according to claim 17, wherein no other monomers are present besides unsaturated compounds of the general formula (I) and acrylic acid and methacrylic acid or acrylic acid and maleic acid (anhydride) or methacrylic acid and maleic acid (anhydride).

21. The polymer according to claim 20, wherein no other monomers are present besides unsaturated compounds of the general formula (I), acrylic acid, methacrylic acid and maleic acid (anhydride).

22. The polymer according to any of claims 7 to 21, wherein from 5 to 99.9% by weight of unsaturated compounds of the general formula (I), (Ia) or (Ib) are present in the polymer, based on the total amount of monomers.

23. The polymer according to any of claims 7 to 21, wherein from 5 to 99.9% by weight of unsaturated compounds of the general formula (I) and from 95 to 0.1% by weight of further, monoethylenically unsaturated monomers are present, in each case based on the total amount of monomers.

24. The polymer according to claim 18, wherein from 5 to 99.9% by weight of unsaturated compounds of the general formula (I) and in total from 95 to 0.1% by weight of acrylic acid, methacrylic acid, (meth)acrylate, maleic acid (anhydride), (iso)prenyl alkoxylate, (meth)allyl alkoxylate or hydroxybutyl vinyl ether alkoxylate are present, in each case based on the total amount of monomers.

25. The polymer according to any of claims 7 to 24, wherein the number-average molecular weight Mₙ of the polymers is 1000 g/mol to 1 000 000 g/mol.

26. The polymer according to any of claims 7 to 24, wherein the number-average molecular weight Mₙ of the polymers is 5000 g/mol to 100 000 g/mol.

27. A method for preparing polymers according to any of claims 7 to 26, wherein unsaturated compounds (I) and optionally at least one further monomer are subjected to free-radical polymerization.

28. The method according to claim 27, wherein said method is a copolymerization in which unsaturated compounds (I) and at least one further monoethylenically unsaturated monomer are subjected to free-radical polymerization.

29. The method according to claim 28, wherein the monoethylenically unsaturated monomers are monomers comprising acid groups, where the acid groups may also be completely or partly neutralized.

30. The method according to any of claims 27 to 29, wherein unsaturated compounds (Ia) are involved.

31. The method according to any of claims 27 to 29, wherein unsaturated compounds (Ib) are involved.

32. The method according to any of claims 27 to 31, wherein the free-radical polymerization is carried out in aqueous solution.

33. The method according to claim 32, wherein the pH in the course of the polymerization is 1 to 6.

34. The method according to claim 32, wherein the pH in the course of the polymerization is 1 to 3.

35. The method according to any of claims 28 to 34, wherein the free-radical polymerization is carried out by initially charging a portion of the unsaturated compounds (I), (Ia) or (Ib), a portion of the further monomers and also a portion of a polymerization initiator in a reactor, and gradually metering into the polymerization reactor the remaining amounts of the unsaturated compounds (I), (Ia) or (Ib), the further monomers and also the polymerization initiator.

36. The method according to claim 35, wherein the amount of monomers initially charged does not exceed 25% by weight of the total amount of the monomers.

37. The use of polymers according to claims 7 to 26 as cement additives, grinding aids in the production of cement, concrete plasticizers, additives to hydraulic binders, reactive plasticizers for preparing plastics, rubber or latex, associative thickeners and antioxidants, or for preparing polyether siloxanes.

38. A mixture comprising polymers according to claims 7 to 26.

## Revendications

1. Composés insaturés de formule générale (I) 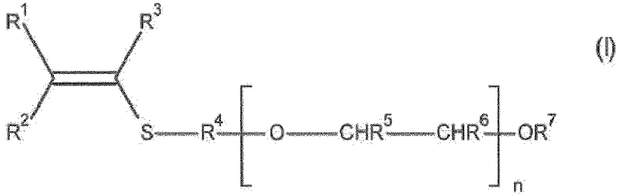 dans laquelle
R¹, R², R³ signifient indépendamment les uns des autres, de manière identique ou différente, H, CH₃,
R⁴ signifie alkylène en C₁-C₃₀ linéaire ou ramifié,
R⁵, R⁶ signifient indépendamment l'un de l'autre, de manière identique ou différente, H, alkyle en C₁-C₂₀, cycloalkyle en C₃-C₁₅, aryle, -CH₂-O-alkyle en C₁-C₂₀, CH₂-O-alcényle en C₂-C₂₀, R⁵ et R⁶ pouvant également former ensemble un alkylène en C₃-C₆,
les R⁷ signifient indépendamment les uns des autres, de manière identique ou différente, H, alkyle en C₁-C₄, 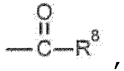
R⁸ signifie alkyle en C₁-C₂₂, alcényle en C₂-C₂₂, et
n signifie un nombre entier de 2 à 200.

2. Composés insaturés selon la revendication 1, **caractérisés en ce que** R⁴ représente un groupe alkylène en C₂-C₄, R⁵, R⁶ représentent indépendamment l'un de l'autre, de manière identique ou différente, un groupe choisi parmi H, -CH₃, -CH₂-CH₃, alkyle en C₃-C₁₁, alkyle en C₁₂-C₂₂, phényle, -CH₂-O-alkyle en C₁-C₁₀, CH₂-O-alcényle en C₂-C₁₀, R⁷ représente H ou alkyle en C₁-C₄, et n représente 5 à 140.

3. Composés insaturés selon la revendication 1, **caractérisés en ce qu'**il s'agit de composés insaturés (Ia) 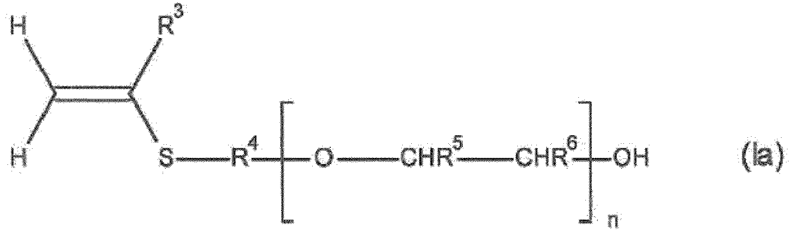 dans laquelle
R³ représente H ou méthyle,
R⁴ représente un groupe alkylène en C₂-C₁₀ linéaire ou ramifié,
R⁵, R⁶ représentent indépendamment l'un de l'autre H, méthyle ou éthyle, à condition que la somme des atomes de carbone dans les radicaux R⁵ et R⁶ par groupe alcoxy soit à chaque fois de 0 à 2, et
n représente 5 à 160.

4. Composés insaturés selon la revendication 1, **caractérisés en ce qu'**il s'agit de composés insaturés (Ib) 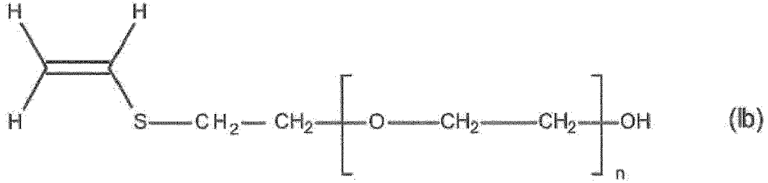 dans laquelle n représente 20 à 140.

5. Composés insaturés selon la revendication 4, **caractérisés en ce que** n représente 20 à 30.

6. Mélanges, contenant des composés de formule générale (I) selon la revendication 1.

7. Polymères, contenant des composés de formule générale (I) selon la revendication 1 en tant que monomères.

8. Polymères selon la revendication 7, contenant des composés de formule générale (Ia) selon la revendication 3 en tant que monomères.

9. Polymères selon la revendication 7, contenant des composés de formule générale (Ib) selon la revendication 4 en tant que monomères.

10. Polymère selon l'une quelconque des revendications 7 à 9, **caractérisé en ce qu'**au moins un autre monomère différent des composés de formule générale (I), (Ib) ou (Ic) est contenu dans le polymère.

11. Polymère selon la revendication 10, **caractérisé en ce que** les autres monomères sont au moins un monomère monoéthyléniquement insaturé.

12. Polymère selon la revendication 11, **caractérisé en ce que** les monomères monoéthyléniquement insaturés sont des monomères comprenant des groupes acides, les groupes acides pouvant également être neutralisés en totalité ou en partie.

13. Polymère selon la revendication 12, **caractérisé en ce que** les groupes acides sont choisis parmi les groupes acide carboxylique, acide sulfonique, acide phosphorique ou acide phosphonique.

14. Polymère selon la revendication 13, **caractérisé en ce que** le polymère comprend au moins un monomère contenant des groupes acide carboxylique choisi dans le groupe constitué par l'acide acrylique, l'acide méthacrylique, l'anhydride de l'acide (méth)acrylique, l'acide crotonique, l'acide maléique, l'anhydride de l'acide maléique, l'acide fumarique, l'acide itaconique, l'acide mésaconique, l'acide citraconique ou l'acide méthylène-malonique ou leurs sels.

15. Polymère selon la revendication 13, **caractérisé en ce que** le polymère comprend au moins un monomère contenant des groupes acide phosphorique ou phosphonique choisi dans le groupe constitué par l'acide vinylphosphonique, les esters de (méth)acrylate d'hydroxyéthyle, d'hydroxypropyle ou d'hydroxybutyle avec de l'acide (poly)phosphorique, l'ester monovinylique de l'acide phosphorique, l'acide allylphosphonique, l'ester monoallylique de l'acide phosphorique, l'acide 3-buténylphosphonique, l'ester mono-3-buténylique de l'acide phosphorique, l'ester mono-(4-vinyloxybutylique) de l'acide phosphorique, l'ester mono-(2-hydroxy-3-vinyloxy-propylique) de l'acide phosphorique, l'ester mono-(1-phosphonoxyméthyl-2-vinyloxy-éthylique) de l'acide phosphorique, l'ester mono-(3-allyIoxy-2-hydroxy-propylique) de l'acide phosphorique, l'ester mono-2-(allylox-1-phosphonoxyméthyl-éthylique) de l'acide phosphorique, le 2-hydroxy-4-vinyloxyméthyl-1,3,2-dioxaphosphol, le 2-hydroxy-4-allyloxyméthyl-1,3,2-dioxaphosphol ou leurs sels.

16. Polymère selon la revendication 13, **caractérisé en ce que** le polymère comprend au moins un monomère contenant des groupes acide sulfonique choisi dans le groupe constitué par l'acide vinylsulfonique, l'acide 2-acrylamido-2-méthylpropanesulfonique, l'acide 2-acrylamidométhyldodécylsulfonique, l'acide 2-(méth)acryloxyéthanesulfonique, l'acide 3-(méth)acryloxypropanesulfonique, l'acide allyloxybenzène-sulfonique, l'acide vinylbenzènesulfonique, l'acide vinyltoluène-sulfonique, l'acide allylsulfonique, l'acide méthallylsulfonique ou leurs sels.

17. Polymère selon l'une quelconque des revendications 7 à 9, **caractérisé en ce que** du styrène, du butadiène, du méthacrylate de méthyle, du (méth)acrylate, de l'acrylate d'éthyle, du maléate de dibutyle, de l'alpha-cyanacrylate de méthyle, de l'acrylonitrile, de l'acide acrylique, de l'acide méthacrylique, de l' (anhydride de l')acide maléique, de l'acide itaconique, de l'acide vinylpbosphonique, de la N-vinylpyrrolidone, du chlorure de N,N-dimétyl-N,N-diallylammonium, de l'acrylamide, du vinylimidazole, de l'alcool vinylique, de l'acétate de vinyle, de l'acide allylsulfonique, de l'acide 2-acrylamido-2-méthylpropane-sulfonique, de l'alcoxylate d'(iso)prényle, de l'alcoxylate de (méth)allyle ou de l'éther-alcoxylate d'hydroxybutylvinyle est contenu en tant qu'au moins un monomère monoéthyléniquement insaturé.

18. Polymère selon la revendication 17, dans lequel de l'acide acrylique, de l'acide méthacrylique, du (méth)acrylate, de l'(anhydride de l')acide maléique, de l'alcoxylate d' (iso)prényle, de l'alcoxylate de (méth)allyle ou de l'éther-alcoxylate d'hydroxybutylvinyle est contenu en tant que monomère monoéthyléniquement insaturé.

19. Polymère selon la revendication 18, dans lequel aucun monomère supplémentaire n'est contenu en plus des composés insaturés de formule générale (I), de l'acide acrylique, de l'acide méthacrylique, du (méth)acrylate, de l' (anhydride de l')acide maléique, de l'alcoxylate d'(iso)prényle, de l'alcoxylate de (méth)allyle ou de l'éther-alcoxylate d'hydroxybutylvinyle.

20. Polymère selon la revendication 17, dans lequel aucun monomère supplémentaire n'est contenu en plus des composés insaturés de formule générale (I) et de l'acide acrylique et de l'acide méthacrylique ou de l'acide acrylique et de l' (anhydride de l') acide maléique ou de l'acide méthacrylique et de l'(anhydride de l') acide maléique.

21. Polymère selon la revendication 20, dans lequel aucun monomère supplémentaire n'est présent en plus des composés insaturés de formule générale (I), de l'acide acrylique, de l'acide méthacrylique et de l' (anhydride de l') acide maléique.

22. Polymère selon l'une quelconque des revendications 7 à 21, **caractérisé en ce que** 5 à 99,9 % en poids de composés insaturés de formule générale (I), (Ia) ou (Ib) sont contenus par rapport à la quantité totale de monomères dans le polymère.

23. Polymère selon l'une quelconque des revendications 7 à 21, **caractérisé en ce que** 5 à 99,9 % en poids de composés insaturés de formule générale (I) et 95 à 0,1 % en poids d'autres monomères monoéthyléniquement insaturés sont contenus, à chaque fois par rapport à la quantité totale de monomères.

24. Polymère selon la revendication 18, dans lequel 5 à 99,9 % en poids de composés insaturés de formule générale (I) et au total 95 à 0,1 % en poids d'acide acrylique, d'acide méthacrylique, de (méth)acrylate, d' (anhydride de l') acide maléique, d'alcoxylate d' (iso)prényle, d'alcoxylate de (méth)allyle ou d'éther-alcoxylate d'hydroxybutylvinyle sont contenus, à chaque fois par rapport à la quantité totale de monomères.

25. Polymère selon l'une quelconque des revendications 7 à 24, **caractérisé en ce que** le poids moléculaire moyen en nombre Mₙ des polymères est de 1 000 g/mol à 1 000 000 g/mol.

26. Polymère selon l'une quelconque des revendications 7 à 24, **caractérisé en ce que** le poids moléculaire moyen en nombre Mₙ des polymères est de 5 000 g/mol à 100 000 g/mol.

27. Procédé de fabrication de polymères selon l'une quelconque des revendications 7 à 26, **caractérisé en ce que** des composés insaturés (I) et éventuellement au moins un autre monomère sont polymérisés par voie radicalaire.

28. Procédé selon la revendication 27, **caractérisé en ce qu'**il s'agit d'une copolymérisation lors de laquelle des composés insaturés (I) et au moins un autre monomère monoéthyléniquement insaturé sont polymérisés par voie radicalaire.

29. Procédé selon la revendication 28, **caractérisé en ce que** les monomères monoéthyléniquement insaturés sont des monomères comprenant des groupes acides, les groupes acides pouvant également être neutralisés en totalité ou en partie.

30. Procédé selon l'une quelconque des revendications 27 à 29, **caractérisé en ce qu'**il s'agit de composés insaturés (Ia).

31. Procédé selon l'une quelconque des revendications 27 à 29, **caractérisé en ce qu'**il s'agit de composés insaturés (Ib).

32. Procédé selon l'une quelconque des revendications 27 à 31, **caractérisé en ce que** la polymérisation radicalaire est réalisée dans une solution aqueuse.

33. Procédé selon la revendication 32, **caractérisé en ce que** le pH au cours de la polymérisation est de 1 à 6.

34. Procédé selon la revendication 32, **caractérisé en ce que** le pH au cours de la polymérisation est de 1 à 3.

35. Procédé selon l'une quelconque des revendications 28 à 34, **caractérisé en ce que** la polymérisation radicalaire est réalisée en chargeant une partie des composés insaturés (I), (Ia) ou (Ib), une partie des autres monomères et une partie d'un initiateur de polymérisation dans un réacteur, et en ajoutant les quantités restantes des composés insaturés (I), (Ia) ou (Ib), des autres monomères et de l'initiateur de polymérisation au fur et à mesure dans le réacteur de polymérisation.

36. Procédé selon la revendication 35, **caractérisé en ce que** la quantité des monomères chargés ne dépasse pas 25 % en poids de la quantité totale des monomères.

37. Utilisation de polymères selon les revendications 7 à 26 en tant qu'additifs pour ciment, adjuvants de broyage lors de la fabrication de ciment, plastifiants pour béton, additifs pour liants hydrauliques, plastifiants réactifs pour la fabrication de plastiques, de caoutchouc ou de latex, épaississants associatifs, antioxydants ou pour la fabrication de polyéthersiloxanes.

38. Mélanges contenant des polymères selon les revendications 7 à 26.
